(19) Europäisches Patentamt
European Patent Office
Office européen des brevets

(11) **EP 3 641 649 B1**

(12) **EUROPEAN PATENT SPECIFICATION**

(45) Date of publication and mention
of the grant of the patent:
**29.11.2023 Bulletin 2023/48**

(21) Application number: **18731115.4**

(22) Date of filing: **20.06.2018**

(51) International Patent Classification (IPC):
**A61B 5/0507** (2021.01)    **G01S 13/58** (2006.01)
**A61B 5/08** (2006.01)    **A61B 5/113** (2006.01)
**A61B 5/00** (2006.01)    **A61B 5/18** (2006.01)
**G01S 13/88** (2006.01)

(52) Cooperative Patent Classification (CPC):
**A61B 5/0507; A61B 5/0816; A61B 5/113;**
**A61B 5/18; G01S 13/586; G01S 13/88;**
A61B 5/0826; A61B 5/6893; A61B 2503/045;
A61B 2503/22

(86) International application number:
**PCT/EP2018/066456**

(87) International publication number:
**WO 2018/234394 (27.12.2018 Gazette 2018/52)**

(54) **SYSTEM AND METHOD FOR BREATHING MONITORING USING RADAR-BASED SENSOR SYSTEMS AND THE SIGNAL AUTOCORRELATION FUNCTION**

SYSTEM UND VERFAHREN ZUR ATEMÜBERWACHUNG UNTER VERWENDUNG VON RADARBASIERTEN SENSORSYSTEMEN UND SIGNALAUTOKORRELATIONSFUNKTION

SYSTÈME ET PROCÉDÉ DE SURVEILLANCE DE RESPIRATION À L'AIDE DE SYSTÈMES DE CAPTEURS BASÉS SUR RADAR ET DE LA FONCTION D'AUTO-CORRÉLATION DE SIGNAL

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **22.06.2017 LU 100324**
**11.09.2017 LU 100438**
**28.12.2017 LU 100633**

(43) Date of publication of application:
**29.04.2020 Bulletin 2020/18**

(73) Proprietor: **IEE International Electronics &**
**Engineering S.A.**
**6468 Echternach (LU)**

(72) Inventors:
• **KARAHASANOVIC, Una**
**54295 Trier (DE)**
• **TATARINOV, Dimitri**
**54296 Trier (DE)**
• **LAMESCH, Laurent**
**8558 Reichlange (LU)**
• **KHAN, Muhammad-Zeeshan**
**88079 Kressbronn (DE)**

(74) Representative: **Office Freylinger**
**P.O. Box 48**
**8001 Strassen (LU)**

(56) References cited:
**US-A1- 2010 152 600**    **US-A1- 2016 200 276**

• **LEE YEE SIONG ET AL: "Detection of respiratory paradoxical movement via Doppler radar measurements", 7TH INTERNATIONAL CONFERENCE ON INFORMATION AND AUTOMATION FOR SUSTAINABILITY, IEEE, 22 December 2014 (2014-12-22), pages 1-5, XP032752810, DOI: 10.1109/ICIAFS.2014.7069602**

## Description

## Technical field

The invention relates to a method of operating a radar sensor system for monitoring a breathing motion of a subject, and to a radar sensor system automatically executing such method.

## Background of the Invention

In the technical field of supporting medical diagnostics of certain breathing diseases or disorders of human beings, such as bronchopulmonary dysplasia, obstructive sleep apnea or upper or lower airway obstruction, it is known to monitor both the chest and the abdomen mechanics. Chest and abdomen undergo periodic motion during breathing but not necessarily in a synchronous manner, a fact that can be characterized by a phase lag $\phi$. A phase lag $\phi = 0$ indicates completely synchronicity, while a phase lag of $\phi = \pi$ indicates that both motions are completely asynchronous. Knowledge about a degree of asynchronicity represented by the phase lag $\phi$ can be exploited for supporting diagnostics. For instance, prematurely born infants or patients with certain neuromuscular diseases can display a rather large asynchronicity between a chest motion and an abdominal motion.

State of the art experimental methods for monitoring breathing and chest-abdomen mechanics are various types of plethysmography. Plethysmography methods, in particular for infants, are described in textbooks, by way of example in "Infant Respiratory Function Testing" by Janet Stocks, Peter D. Sly, Robert S. Tepper and Wayne J. Morgan (editors), John Wiley & Sons, 1996 (ISBN: 978-0-471-07682-7).

Plethysmography methods, in particular when applied to infants and children, may suffer from quite large uncertainties and errors in the phase lag $\phi$ between the chest and abdominal motion.

Further, it has been proposed to measure the degree of a chest-abdomen asynchronicity using radar Doppler measurements as a contactless method.

In the article "Non-contact diagnostic system for sleep apnea-hypopnea syndrome based on amplitude and phase analysis of thoracic and abdominal Doppler radars" by Masayuki Kagawa et al., a non-contact sleep apnea-hypopnea syndrome (SAHS) diagnostic system is described that can detect apneic events without inducing stress in monitored individuals. Two Doppler radars are installed beneath the mattress to measure the vibrations of the chest and abdomen, respectively. The SAHS determines apnea and hypopnea events when the radar output amplitude decreases by <20 and 70%, respectively, of the amplitude of a normal breath (without SAHS events). Additionally, a technique is proposed that detects paradoxical movements by focusing on phase differences between thoracic and abdominal movements, and are able to identify three types of sleep apnea: obstructive, central, and mixed. Respiratory disturbance indexes obtained showed a higher correlation (r = 94%) with full-night polysomnography (PSG), the recognized gold standard test, than with pulse oximetry (r = 89%). When predicting the severity of SAHS with an apnea-hypopnea index (AHI) of >15/h or >30/h using PSG as a reference, the radar system achieved a sensitivity of 96 and 90%, and a specificity of 100 and 79% with an AHI of >15/h and >30/h, respectively.

In the article by Y. S. Lee, P. N. Pathirana and C. L. Steinfort: "Detection of respiratory paradoxical movement via Doppler radar measurements", 7th International Conference on Information and Automation for Sustainability, Colombo, 2014, pp. 1-5 (doi: 10.1109/ICIAFS.2014.7069602), an evaluation of microwave Doppler radar in capturing the respiration signal from the chest and abdomen simultaneously using two radar systems is described. Two experiments were conducted to investigate the feasibility of using Doppler radar in measuring respiration from both chest and abdomen simultaneously. Results obtained indicate that the respiration patterns from the radar were highly correlated with the reference respiration strap readings for normal breathing scenarios and also sensitive enough in capturing the paradoxical movement between the chest and the abdomen in the professionally role played experiments.

In patent application US 2016/0200276 A1 a system and method are proposed for sensing occupancy status within an automotive vehicle using a radar sensor system. The radar sensor system comprises an antenna system, at least one sensor and processing circuitry. The method comprises illuminating, using the antenna system, at least one occupiable position within the vehicle with continuous wave (CW) signals, the CW signals being frequency modulated in time. At least one sensor signal a reflected as a result of the CW signals is received using the at least one sensor. The at least one sensor defines a plurality of receive channels 1... i, each channel having a different frequency f1 ...fi. Processing circuitry is operable for applying, for each receive channel 1... i DC offset removal to the corresponding sensor signal a to generate a modified signal b, and generating, based on the modified signals b one or more occupancy status signals. The occupancy status signal indicates a property related to the at least one occupiable position. A system for carrying out the method is also disclosed. The techniques provide for in-vehicle occupant detection and classification (airbag suppression), for passenger presence detection and passenger's vital sign monitoring and for seatbelt reminder functionality (SBR). The radar system may consist of one, two or four outputs I/Q per channel. The received signal may be mixed down with a single channel demodulator or an I/Q demodulator into the baseband. An advanced signal processing including an auto calibration routine, and clutter removal may be used to detect occupants and their vital sign (breathing and heart beat) and to determine their distance to the radar system.

International application WO 2015/140333 A1 describes a method for ascertaining whether an unattended child is present within an automotive vehicle, using a radar sensor system comprising a transmitter and at least one sensor and processing circuitry and exploiting a breathing motion detected by radar signals, for instance by applying autocorrelation and peak finding. The method comprises: illuminating at least one occupiable position within the vehicle with radiation, the radiation exhibiting multiple frequencies; generating radar sensor signals from radiation reflected as a result of the transmitted radiation, a plurality of the radar sensor signals corresponding to different frequencies; operating the processing circuitry for generating, based on the radar sensor signals, a first indicator value, the first indicator value indicating a degree of motion associated with the occupiable position; determining whether the first indicator value satisfies a first predetermined criteria; if the first indicator value satisfies the first predetermined criteria, generating, based on radar sensor signals, a second indicator value, the second indicator value indicating a degree of repetitive pattern within the radar sensor signals; and determining that an unattended child is present within the automotive vehicle if the second indicator value satisfies a second predetermined criteria. The second indicator value may comprise a breathing signature indicative of the extent to which the sensor signals indicate that motion indicative of infant breathing child is detected.

In the article "Non-Contact Estimation at 60 GHz for Human Vital Signs Monitoring Using a Robust Optimization Algorithm" by Ting Zhang et al., Conference IEEE APS 2016, Jun 2016, Fajardo (Porto-Rico), United States, 2016, AP-S/URSI 2016. <hal-01340613>, an approach to estimate body movements related to vital activities by means of a 60 GHz Doppler radar is described, using robust optimization algorithms including signal autocorrelation analysis in order to extract heart-rate and breathing information from the radar signals.

Patent application US 2010/0152600 A1 describes a radar-based physiological motion sensor. Doppler-shifted signals can be extracted from the signals received by the sensor. The Doppler-shifted signals can be digitized and processed subsequently to extract information related to the cardiopulmonary motion in one or more subjects. The information can include respiratory rates, heart rates, waveforms due to respiratory and cardiac activity, direction of arrival, abnormal or paradoxical breathing, etc. Information regarding an irregularity or regularity of respiration can include assessment of whether irregular breathing is periodic. This assessment can include estimating each breath-to-breath interval, and storing it with the time point at the end of the interval in which it was calculated; interpolating between these breath-to-breath intervals to create a waveform; performing the Fourier transform, performing the autocorrelation function, or calculating the power spectral density of the waveform; determining whether there are significant peaks of the Fourier transform, the autocorrelation function, or the power spectral density of the waveform; and determining that if significant peaks exist, the breathing is irregular and periodic.

**Object of the invention**

It is desirable to provide a non-contact method for reliably and quantitatively monitoring a breathing motion of a subject, particularly a human being.

It is therefore an object of the invention to provide a method for monitoring a breathing motion of a subject, particularly of a human being, that is capable of extracting and evaluating a quantity of one or more breathing-characteristic parameters from signals that are acquired from the subject by sensors operating in a contactless manner. The invention is defined by appended claims 1-14.

**General Description of the Invention**

The present invention is particularly useful as a supporting tool for medical purposes such as diagnostics, as a monitoring device for automotive applications such as advanced driver assistance systems (ADAS), or as a monitoring device in sports.

In one aspect of the present invention, the object is achieved by a method of operating a radar sensor system for monitoring a breathing motion of a subject. The radar sensor system includes a radar transmitting unit having one radar transmitting antenna and being configured for transmitting radar waves towards the subject, a radar receiving unit having one radar receiving antenna and being configured for receiving radar waves that have been transmitted by the radar transmitter unit and have been reflected by the subject, and an evaluation and control unit that is at least configured for evaluating Doppler information from the radar waves received by the radar receiving unit.

The method comprises at least steps of

- operate the radar transmitting unit for at least once transmitting radar waves of a predetermined radar carrier frequency for a specified time towards a chest and an abdominal region of the subject,
- operate the radar receiving unit at least once for a specified time for receiving radar waves that have been transmitted by the radar transmitting unit and that have been reflected by the chest and the abdominal region of the subject, and for generating received radar signals from the received radar waves,

- calculate the autocorrelation function of the received radar signals,
- calculate the Fourier transform of the calculated autocorrelation function,
- determine and record values of at least two peaks of the calculated Fourier transform,
- change the number of radar wavelengths in the path between the radar transmitting antenna, the subject (24) and the radar receiving antenna at least once by a predetermined amount,
- iteratively carry out the preceding steps until at least one extreme value is detected for the determined values of each one the at least two peaks, and
- from the recorded determined and detected extreme values of the at least two peaks, determine at least one breathing-characteristic parameter of the breathing motion of the subject.

The phrase "being configured to", as used in this application, shall in particular be understood as being specifically programmed, laid out, furnished or arranged.

The phrase "evaluating Doppler information", as used in this application, shall in particular be understood as evaluating received radar waves for ranging purposes.

The at least one breathing-characteristic parameter of the breathing motion of the subject may be given by, without being limited to, a chest breathing amplitude and an abdominal region breathing amplitude.

One advantage of the proposed method lies in that a non-contact measurement is provided that does not disturb the subject and avoids to potentially influence a result of the desired measurement.

Another advantage lies in that only one radar transmitter unit and only one radar receiving unit is required, resulting in a part and cost saving solution, which moreover reduces any necessary synchronization effort.

Further, by using the signal autocorrelation function, random noise beneficially cancels out, enabling more precise measurements in suitable embodiments, for instance of the phase lag between breathing motions of the chest and the abdominal region of the subject. This means an improvement in particular compared to the well-known in-phase or quadrature signal processing in this technical field.

Further, with the proposed method, a chest breathing amplitude and/or an abdominal region breathing amplitude can be determined with low error of measurement. This benefit can in particular be also achieved if the breathing-characteristic parameter is formed by a phase lag between breathing motions of the chest and the abdominal region of the subject.

By using the proposed method, diagnostics of patients with certain neuromuscular diseases and prematurely born infants can be supported. Moreover, the proposed method can enable to identify if a person is undergoing pulmonary or diaphragmatic breathing, which may be useful for many applications.

Preferably, the evaluation and control unit comprises a processor unit and a digital data memory unit to which the processor unit has data access. In this way, the steps of recording received radar signals and the calculating steps can be executed within the radar sensor system to enable a fast and undisturbed signal processing and evaluation.

In some embodiments, the radar transmitting unit and the radar receiving unit may be designed as distinct units. In other embodiments, the radar transmitting unit and the radar receiving unit may be designed as a transceiver unit, having common electronic circuitry and/or a common housing.

In some preferred embodiments of the method, the step of changing the number of radar wavelengths in the path includes carrying out a predetermined displacement along a predetermined direction of at least one out of the radar transmitting antenna and the radar receiving antenna relative to the subject. In this way, the number of radar wavelengths in the path can be changed in a constructively simple manner. For instance, the predetermined direction may be chosen as a straight line pointing essentially towards the subject and forming an acute angle with an angle bisector of an angle formed by the chest, the radar sensor system as the vertex and the abdominal region of the subject.

In some preferred embodiments of the method, the step of changing the number of radar wavelengths in the path includes changing a radar carrier frequency of the radar sensor system by a predetermined amount. By that, a fast, simple and precise change of the number of radar wavelengths in the path can be enabled, for instance by tuning a control voltage at a control input line of a voltage controlled oscillator (VCO) of the radar transmitting unit.

For such embodiments of the method, the step of operating the radar transmitting unit for transmitting radar waves preferably includes transmitting a plurality of consecutive radar wave trains, each wave train comprising a plurality of radar waves of predetermined duration, wherein radar carrier frequencies of each radar wave of the plurality of radar waves differ by an integral multiple of the predetermined amount. This feature is a variation of a concept commonly known as Frequency Shift Keying Radar. Further, the step of operating the radar receiving unit for receiving radar waves includes receiving radar waves that have been reflected by the chest and abdominal region of the subject for each radar wave of the plurality of radar waves and for each radar wave train of the plurality of radar wave trains, and includes generating and recording received radar signals for each one of the received radar waves. In a suitable embodiment, the method can thus allow for a shortened total measurement time.

The consecutive radar wave trains of the plurality of consecutive radar wave trains may be separated by a time gap. Alternatively, the consecutive radar wave trains of the plurality of consecutive radar wave trains may directly follow each other without any time gap.

Preferably, the step of changing the number of radar wavelengths in the path is carried out until at least one minimum value and one maximum value is recorded for the determined values of each one of the at least two peaks of the calculated Fourier transform. In this way, the phase lag between breathing motions of the chest and the abdominal region of the subject and/or the chest breathing amplitude and/or the abdominal region breathing amplitude can be determined from the determined values of each one of the at least two peaks with a smaller error of measurement.

The step of determining at least one breathing-characteristic parameter of the breathing motion of the subject can further include correcting the at least one extreme value for the determined values of one of the at least two peaks by an offset value determined from the recorded determined values of the one of the at least two peaks. In this context it should be noted that the offset alone (as well as the size of the oscillation) carries information (i.e. depends on) the breathing amplitude. To get the size of the oscillation, it is preferable to subtract the offset. But in addition, the offset itself carries info about the breathing amplitudes. It follows that the step of determining a chest breathing amplitude or an abdominal region breathing amplitude can further include evaluating information contained in said offset value.

In such embodiments of the method, in which at least one extreme value is detected for the determined values of each one of the at least two peaks, the step of determining and recording values of the at least two peaks of the calculated Fourier transform includes determining and recording values of a peak of order n and values of a peak of order -n of the calculated Fourier transform, and the step of determining at least one breathing-characteristic parameter of the breathing motion of the subject includes determining a phase lag between breathing motions of the chest and the abdominal region of the subject by determining a difference of the radar carrier frequencies at which extreme values of the peak of order n and the peak of order -n occur.

The phrase "peak of order $\pm n$", as used in this application, shall in particular be understood as a signal peak of the Fourier transform that is located at Fourier frequency $\pm n \cdot f_b$, wherein $f_b$ denotes the breathing frequency.

This is based on the insight that extreme values of the peak of order n and the peak of order -n will only occur for the same value of the radar carrier frequency if the phase lag equals zero. A measured radar carrier frequency difference between extreme values of the peak of order n and the peak of order -n can thus be exploited for determining the phase lag.

Preferably, the step of determining and recording values of at least two peaks of the calculated Fourier transform comprises determining and recording a plurality of at least two additional peaks of the calculated Fourier transform representing higher order harmonics with respect to the at least two peaks, and the step of determining at least one breathing-characteristic parameter of the breathing motion of the subject comprises determining at least one out of determining a chest breathing amplitude and an abdominal region breathing amplitude, and further comprises determining a standstill of the at least one out of a chest breathing amplitude and an abdominal region breathing amplitude by comparing recorded values of the plurality of at least two additional peaks with predetermined threshold values for the plurality of at least two additional peaks, and by detecting that recorded values of the plurality of at least two additional peaks exceed the predetermined threshold values for a predetermined period of time.

This embodiment of the proposed method is particularly beneficial for the application of apnea detection. Apnea is a sudden stop in breathing, in which case a received radar signal will suddenly drop to zero. As a result, higher order frequency harmonics will arise in the Fourier transform of the signal autocorrelation function, which would normally not be present for any realistic values of observable breathing displacement amplitudes. Mathematically speaking, a sharp breathing stop produces step-like signatures in in-phase and quadrature radar signals, which is equivalent to a larger number of higher order frequency harmonics in the Fourier space. A detected occurrence of higher order frequency harmonics in the Fourier space can thus be used as an apnea alarm trigger.

Preferably, a smallest gap between the at least two peaks and the plurality of at least two additional peaks of the calculated Fourier transform is equivalent to at least two orders, more preferably to at least three orders, and, most preferably, to at least four orders. In this way, an especially robust apnea detection can be achieved.

In preferred embodiments of the method, the step of determining and recording values of peaks comprises determining and recording values of at least three peaks of the calculated Fourier transform, and the step of determining at least one breathing-characteristic parameter of the breathing motion of the subject includes determining one out of a plurality of predetermined breathing amplitude intervals based on carrying out a ranking of the determined values of the at least three peaks of the calculated Fourier transform.

This is based on the insight that by ranking the determined values of the at least three peaks of the calculated Fourier transform it can be exploited that specific rankings of the values of the at least three peaks can exist in disjoint intervals, by which a chest breathing amplitude and/or an abdominal region breathing amplitude can be determined within specified limits.

Preferably, the at least three peaks of the calculated Fourier transform are of consecutive order. In this way, suitable intervals for breathing amplitudes can be achieved.

Preferably, the step of determining and recording values of peaks comprises determining and recording values of all peaks of the calculated Fourier transform that are larger than a predetermined value. In this way, an especially robust method of estimating a chest breathing amplitude and/or an abdominal region breathing amplitude can be provided.

Preferably, in embodiments of the method in which the step of determining at least one breathing-characteristic parameter of the breathing motion of the subject includes determining a phase lag ($\phi$), the method further comprises steps of

- comparing the determined at least one out of phase lag, chest breathing amplitude and abdominal region breathing amplitude with at least one predetermined threshold value, and
- generating an output signal representing a breathing status of the subject.

By that, a fast evaluation of the measurement can be accomplished that can be exploited for alarm purposes.

In another aspect of the invention, a radar sensor system is provided for monitoring a breathing motion of a subject. The radar sensor system comprises

- a radar transmitting unit having one radar transmitting antenna and being configured for transmitting radar waves of a predetermined radar carrier frequency towards a chest and an abdominal region of the subject,
- a radar receiving unit having one radar receiving antenna and being configured for receiving radar waves that have been transmitted by the radar transmitter unit and have been reflected by the subject, and
- an evaluation and control unit that is at least configured for evaluating Doppler information from the radar waves received by the radar receiving unit, and that is further configured to automatically execute steps of the method as disclosed herein.

The benefits described in context with the method of operating a radar sensor system for monitoring a breathing motion of the subject apply to the proposed radar sensor system automatically executing such method to the full extent.

Preferably, the predetermined radar carrier frequency lies in the frequency range between 15 GHz and 130 GHz, and more preferably in the frequency range between 57 GHz and 64 GHz. This can enable that the bandwidth of components of the radar sensor system is sufficiently large to ensure that at least one extreme value is detected for the determined values of each one of the at least two peaks of the calculated Fourier transform. What is beneficial for, for example a 60 GHz system, is that a rather large bandwidth of 8 GHz is available. Compared to lower radar carrier frequencies, the proposed frequency range can further have the advantage that higher order peaks of the calculated Fourier transform become more pronounced, which can result in a lower error of measurement.

If the radar transmitting antenna is configured to have a main lobe that is able to cover a major part of the chest and the abdominal region of the subject, a radar sensor system for monitoring a breathing motion of a subject with an especially simple construction and operation can be provided. The phrase "major part", as used in this application, shall in particular be understood as more than 30%, preferably more than 50% and, more preferably, more than 60% of an outer surface of the respective region.

In yet another aspect of the invention, a software module for controlling automatic execution of the method disclosed herein is provided.

The method steps to be conducted are converted into a program code of the software module, wherein the program code is implementable in a digital memory unit of the radar sensor system or a separate control unit and is executable by a processor unit of the radar sensor system or a separate control unit. Preferably, the digital memory unit and/or processor unit may be a digital memory unit and/or a processing unit of the control and evaluation unit of the radar sensor system. The processor unit may, alternatively or supplementary, be another processor unit that is especially assigned to execute at least some of the method steps.

The software module can enable a robust and reliable automatic execution of the method and can allow for a fast modification of method steps.

These and other aspects of the invention will be apparent from and elucidated with reference to the embodiments described hereinafter.

It shall be pointed out that the features and measures detailed individually in the preceding description can be combined with one another in any technically meaningful manner and show further embodiments of the invention. The description characterizes and specifies the invention in particular in connection with the figures.

**Brief Description of the Drawings**

Further details and advantages of the present invention will be apparent from the following detailed description of not limiting embodiments with reference to the attached drawing, wherein:

Fig. 1 schematically illustrates a configuration of an embodiment of the radar sensor system in accordance with the invention while executing an operating method for monitoring a breathing motion of a subject,

Fig. 2a shows the calculated Fourier transform of the autocorrelation function of radar waves received in the configuration pursuant to Fig. 1,

Fig 2b shows another calculated Fourier transform of an autocorrelation function of radar waves received during an apnea event in the configuration pursuant to Fig. 1,

Fig. 3 shows a plot of the variation of amplitudes of two adjacent peaks of the calculated Fourier transform pursuant to Fig. 2a as a result of predetermined displacements of the radar sensor system pursuant to Fig. 1,

Fig. 4 shows a plot of the variation of amplitudes of two adjacent peaks of the calculated Fourier transform pursuant to Fig. 2a as a result of predetermined changes of the radar carrier frequency of the radar sensor system pursuant to Fig. 1,

Fig. 5 shows a plot of values of the first five peaks of another calculated Fourier transform of an autocorrelation function of other received radar waves vs. a breathing amplitude for a fixed radar carrier frequency,

Fig. 6 shows a plot of the variation of values of a peak of order n and values of a peak of order -n of the calculated Fourier transform of an autocorrelation function of other received radar waves for two distinct values of a phase lag,

Fig. 7 is a flowchart of an embodiment of the method in accordance with the invention of operating the radar sensor system pursuant to Fig. 1 for monitoring a breathing motion of a subject,

Fig. 8 is a flowchart of an alternative embodiment of the method in accordance with the invention of operating the radar sensor system pursuant to Fig. 1 for monitoring a breathing motion of a subject,

Fig. 9 is a flowchart of another alternative preferred embodiment of a method in accordance with the invention of operating the radar sensor system pursuant to Fig. 1 for monitoring a breathing motion of a subject,

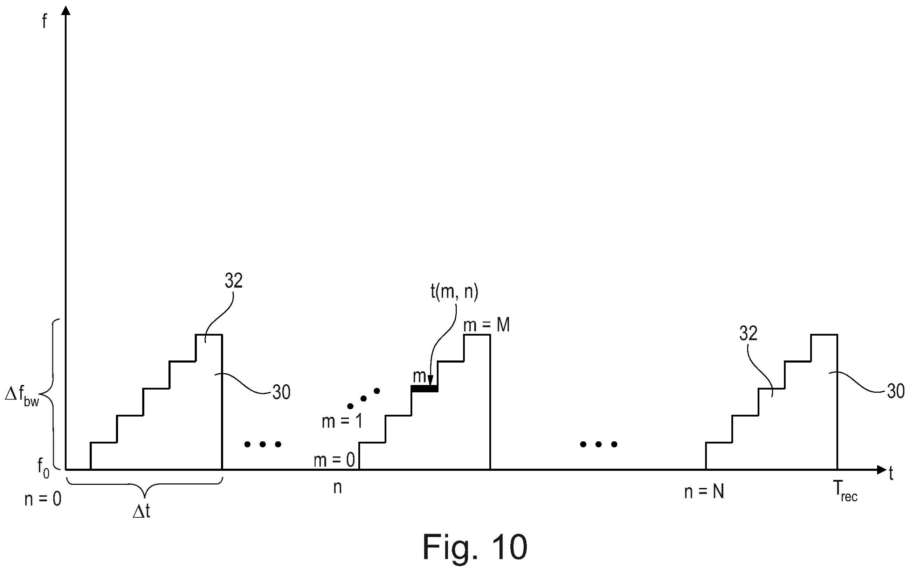

Fig. 10 illustrates the plurality of consecutive radar wave trains of the fast frequency sweep of the radar carrier frequency as executed in a step of the method pursued to Fig. 7, and

Fig. 11 shows the simulated Fourier transform of the autocorrelation function of yet another possible embodiment.

## Description of Preferred Embodiments

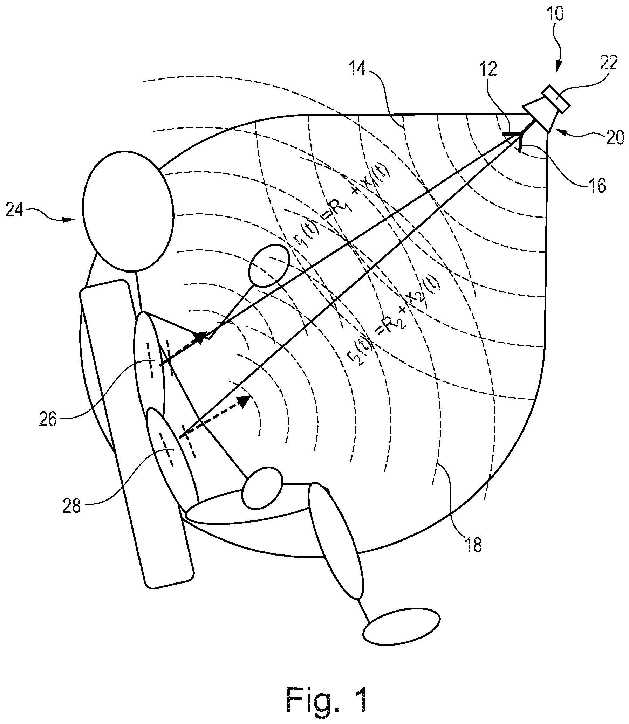

Fig. 1 schematically illustrates a configuration of an embodiment of the radar sensor system 10 in accordance with the invention while executing an operating method for monitoring a breathing motion of a subject 24.

The radar sensor system 10 comprises a radar transmitting unit having one radar transmitting antenna 12 and being configured for transmitting radar waves 14 of a predetermined radar carrier frequency towards a chest 26 and an abdominal region 28 of the subject 24. The radar transmitting antenna 12 is directed towards the subject 24 and is designed to have a main lobe that is able to cover a major part of more than 50% of the chest 26 and the abdominal region 28 of the subject 24. The predetermined radar carrier frequency is controllable within a frequency range between 15 GHz and 130 GHz, and more preferably in a frequency range between 57 GHz and 64 GHz, corresponding to wavelengths $\lambda$ in air between 5.3 mm and 4.7 mm.

The radar sensor system 10 further includes a radar receiving unit having one radar receiving antenna 16 and being configured for receiving radar waves 18 that have been transmitted by the radar transmitter unit and have been reflected by the subject 24.

The radar transmitting antenna 12 and the radar receiving antenna 16 are co-located in a monostatic arrangement, which is indicated in Fig. 1 by use of a combined symbol. In this specific embodiment, the radar transmitter unit and the radar receiving unit form an integral part of a transceiver unit 20, sharing common electronic circuitry and a common housing. In other embodiments, the radar transmitter unit and the radar receiving unit may be designed as separate units.

Moreover, the radar sensor system 10 comprises an evaluation and control unit 22 that is configured for evaluating Doppler information from the radar waves 18 received by the radar receiving unit. The evaluation and control unit 22 is connected to the radar transmitting unit for controlling operation of the radar transmitting unit. The evaluation and control unit 22 is also connected to the radar receiving unit for receiving radar signals generated by the radar receiving unit. The evaluation and control unit 22 comprises a processor unit and a digital data memory unit (not shown) to which the processor unit has data access. The evaluation and control unit 22 is configured for recording the received radar signals generated by the radar receiving unit in the digital data memory unit.

The radar sensor system 10 is mechanically connected to a mechanism (not shown) that allows, by control of the control and evaluation unit 22, for a predetermined displacement of the radar sensor system 10 along the predetermined direction relative to the subject 24. A maximum displacement of the mechanism is 10.0 mm.

In a modeling approach, the configuration illustrated in Fig. 1 can mathematically be described as follows.

For simplicity, sinusoidal motion of the chest 26 and the abdominal region 28 of the subject 24 is assumed, although the analysis is applicable for an arbitrary continuous and periodic breathing pattern on grounds of the Fourier theorem, as will be readily acknowledged by those skilled in the art. The chest 26 and the abdominal region 28 undergo periodic sinusoidal motion about some equilibrium positions $R_1$ and $R_2$, which are respective distances to the radar sensor system 10 as shown in Fig. 1. As described before, the abdominal region 28 does not necessarily undergo the identical oscillatory motion as the chest 26. Rather, its motion can be characterized by some phase lag $\phi$ with respect to that of the chest 26. The frequency of the motion of the chest 26 and abdominal region 28 is denoted by $f_b$, and the radial projections of the amplitude of the motion of the chest 26 and the abdominal region 28 by $x_1^0$ and $x_2^0$, respectively. Then, their respective distances $r_1(t), r_2(t)$ to the radar sensor system 10 change with time t according to

$$r_1(t) = R_1 + x_1(t),$$

$$r_2(t) = R_2 + x_2(t), \qquad\qquad\qquad \text{Eq. (1)}$$

with

$$x_1(t) = x_0^1 \ g(2\pi f_b t + \phi),$$

$$x_2(t) = x_0^2 \ g(2\pi f_b t), \qquad\qquad\qquad \text{Eq. (2)}$$

wherein g denotes an arbitrary continuous and periodic function with period $f_b^{-1}$, representing the breathing pattern. This reduces to

$$x_1(t) = x_0^1 \sin(2\pi f_b t + \phi),$$

$$x_2(t) = x_0^2 \sin(2\pi f_b t),$$

in the case of a sinusoidal breathing pattern.

The reflected radar signal is now given by the superposition

$$u_r = A_1 e^{-i(2\pi f t + \frac{4\pi}{\lambda} r_1(t))} + A_2 e^{-i(2\pi f t + \frac{4\pi}{\lambda} r_2(t))}, \qquad\qquad \text{Eq. (3)}$$

wherein $A_1$ and $A_2$ denote the power amplitudes, which depend on

-  the equilibrium distances $R_{1/2}$ in the limit of $x_{1/2}^0 \ll R_{1/2}$, which is always the case for any practical set-up,
-  the antenna gain pattern, and
-  the radar cross sections of the chest 26 and the abdominal region 28, respectively.

In Eq. (3), f denotes the predetermined radar carrier frequency.

After mixing and going through the low-pass filter, which removes the high radar carrier frequency component f, the complex radar signal Y reads

$$Y(t) = B_1 e^{-i\left(\frac{4\pi}{\lambda} r_1(t)\right)} + B_2 e^{-i\left(\frac{4\pi}{\lambda} r_2(t)\right)}, \qquad \text{Eq. (4)}$$

wherein $B_k$, $k$ = 1, 2 represents the signal amplitudes after mixing and the low-pass filter.

In the following, various possible embodiments of a method in accordance with the invention of operating the radar sensor system 10 pursuant to Fig. 1 for monitoring a breathing motion of the subject 24 will be presented. The individual alternative embodiments are identified by a prefix cipher of the particular embodiment, starting with cipher 1. Steps whose function is the same or basically the same in all embodiments are identified by reference numbers made up of the prefix cipher of the embodiment to which it relates, followed by the numeral of the feature.

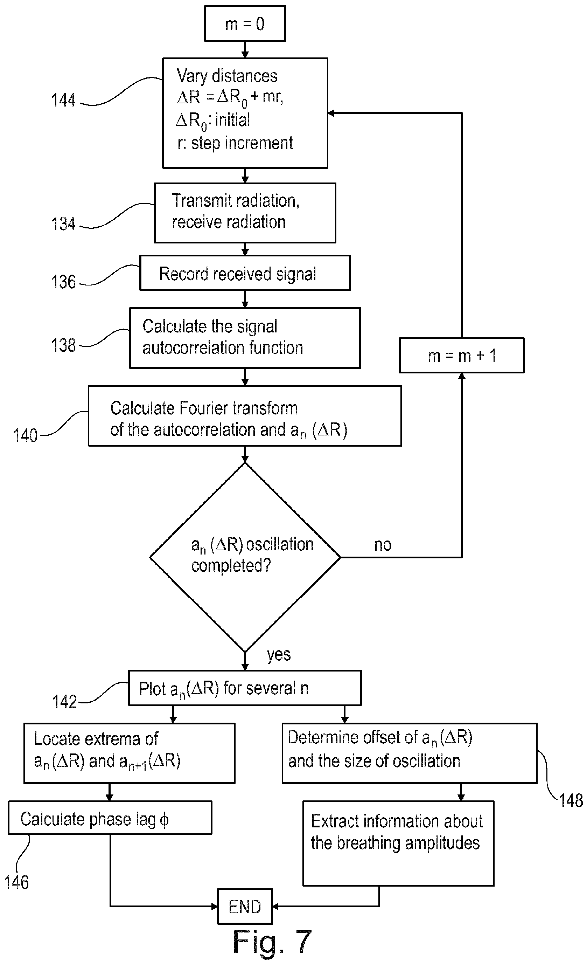

A first embodiment of the method of operating the radar sensor system 10 pursuant to Fig. 1 for monitoring a breathing motion of the subject 24 will be described with reference to Fig. 1. A flowchart of the method is provided in Fig. 7. In preparation of operating the radar sensor system 10, it shall be understood that all involved units and devices are in an operational state and configured as illustrated in Fig. 1.

In order to be able to carry out the method automatically and in a controlled way, the evaluation and control unit 22 comprises a software module. The method steps to be conducted are converted into a program code of the software module. The program code is implemented in the digital data memory unit of the evaluation and control unit 22 and is executable by the processor unit of the evaluation and control unit 22.

With the radar sensor system 10 arranged at a fixed predetermined position relative to the subject 24, the radar transmitting unit is controlled by the control of evaluation unit 22 for transmitting radar waves of the predetermined radar carrier frequency for a specified duration towards the chest 26 and the abdominal region 28 of the subject 24 in a step 134 of the method.

The radar receiving unit receives radar waves that have been transmitted by the radar transmitting unit and that have been reflected by the chest 26 and the abdominal region 28 of the subject 24, and the radar receiving unit generates received radar signals from the received radar waves. In another step 136, the generated received radar signals are received and recorded by the control and evaluation unit 22.

In another step 138 of the method, the autocorrelation function of the received radar signal is calculated.

The autocorrelation function of the complex radar signal $Y$ given by Eq. (4) is defined as

$$A(\tau) = \int_{-\infty}^{\infty} Y(t) \, Y^*(t + \tau) \, dt, \qquad \text{Eq. (5)}$$

wherein $Y^*$ denotes the complex conjugate of the complex radar signal $Y$. The complex signal $Y$ is given by $Y = I + iQ$, where $I$ is the in-phase signal, and $Q$ the quadrature signal, and $i$ the imaginary unit.

In a next step 140, the Fourier transform $A(v)$ of the calculated autocorrelation function $A(\tau)$ is calculated. The letter $v$ denotes the Fourier transform frequency.

The autocorrelation function $A(\tau)$ is a convolution of the complex radar signal $Y$ with itself; therefore the convolution theorem can be applied to calculate the Fourier transform $A(v)$ of the autocorrelation function $A(\tau)$ from

$$A(v) = Y(v) \, Y^*(v), \qquad \text{Eq. (6)}$$

wherein $Y(v)$ is the Fourier transform of the complex radar signal $Y(t)$ given by Eq. (4), i.e.

$$Y(v) = \int_{-\infty}^{\infty} e^{-i2\pi v t} \, Y(t) dt \qquad \text{Eq. (7)}$$

Evaluating the following Fourier transform:

$$\int_{-\infty}^{\infty} e^{-i2\pi v t} e^{-i\frac{4\pi x_0^1 \, g(2\pi f_b t + \phi)}{\lambda}} dt$$

yields

$$= \int_{-\infty}^{\infty} e^{-i2\pi v(\tilde{t}-\frac{\phi}{2\pi f_b})} e^{-i\frac{4\pi x_0^1 \, g(2\pi f_b \tilde{t})}{\lambda}} d\tilde{t}$$

$$= e^{-i\frac{v}{f_b}\phi} \int_{-\infty}^{\infty} e^{-i2\pi v\tilde{t}} e^{-i\frac{4\pi x_0^1 \, g(2\pi f_b \tilde{t})}{\lambda}} d\tilde{t}, \qquad \text{Eq. (8)}$$

wherein a change of variables $\tilde{t} = t + \frac{\phi}{2\pi f_b}$ has been performed in the second line of the above.

Further, one can make the following decomposition according to Eq. (9), which in more detail is described in W. A. Gardner, "Statistical Spectral Analysis: A Nonprobabilistic Theory", Prentice-Hall, Englewood Cliffs, NJ, 1987, and in the cited article "Non-Contact Estimation at 60 GHz for Human Vital Signs Monitoring Using a Robust Optimization Algorithm" by Ting Zhang et al. Both these documents shall hereby be incorporated by reference in their entirety with effect for those jurisdictions permitting incorporation by reference.

$$\int_{-\infty}^{\infty} e^{-i2\pi v\tilde{t}} e^{-i\frac{4\pi x_0^1 \, g(2\pi f_b \tilde{t})}{\lambda}} d\tilde{t} = \sum_{n=-\infty}^{\infty} J_n\left(\frac{4\pi}{\lambda}x_0^1\right)\delta(v - nf_b). \qquad \text{Eq. (9)}$$

Herein, $J_n$ is a basis function, which in principle depends on the shape of the breathing signal (e.g. sinusoidal, triangular, etc.). In case that the function $g$ is the sinus function (i.e. breathing pattern has sinusoidal shape), the function $J_n$ is the nth order spherical Bessel function of the first kind.

Thus, the Fourier transform $Y(v)$ of the complex radar signal $Y(t)$ in Eq. (4) reads

$$Y(v) = B_1 e^{-i\frac{4\pi}{\lambda}R_1}\sum_{n=-\infty}^{\infty} J_n\left(\frac{4\pi}{\lambda}x_0^1\right)e^{-i\frac{v}{f_b}\phi}\delta(v - nf_b) + B_2 e^{-i\frac{4\pi}{\lambda}R_2}\sum_{n=-\infty}^{\infty} J_n\left(\frac{4\pi}{\lambda}x_0^2\right)\delta(v - nf_b)$$

$$\text{Eq. (10)}$$

Using Eq. (6) in combination with the result of Eq. (10), the following can be obtained:

$$A(v) = \sum_{n=-\infty}^{\infty} a_n \, \delta(v - nf_b), \qquad \text{Eq. (11a)}$$

with

$$a_n(f) = C_n(f) + K_n(f)\cos\left(n\phi + \frac{4\pi}{c}f(R_1 - R_2)\right), \qquad \text{Eq. (11b)}$$

$$C_n(f) = B_1^2 J_n^2\left(\frac{4\pi x_1^0}{c}f\right) + B_2^2 J_n^2\left(\frac{4\pi x_2^0}{c}f\right), \text{ and}$$

$$K_n(f) = 2B_1 B_2 J_n\left(\frac{4\pi x_1^0}{c}f\right)J_n\left(\frac{4\pi x_2^0}{c}f\right),$$

wherein $f$ denotes the radar carrier frequency. Thereby, it is proven that Eq. (11a) is applicable for an arbitrarily shaped periodic signal, which is also confirmed by simulations, as will be described thereinafter.

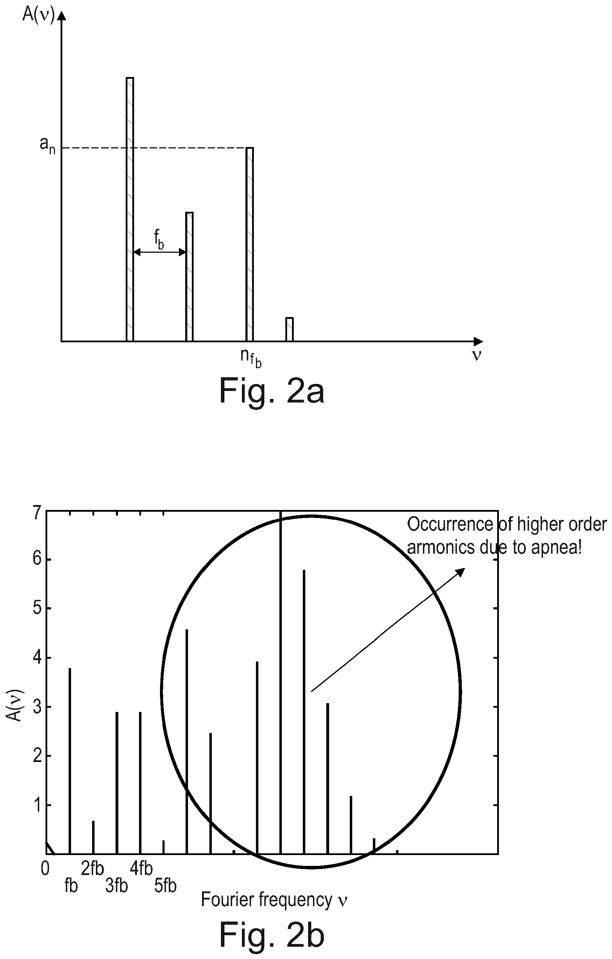

According to Eq. (11a), the Fourier transform $A(v)$ of the autocorrelation function $A(\tau)$ is given by a series of peaks of different heights, separated by $f_b$, the frequency of the motion of the chest and abdominal region of the subject. This is exemplarily illustrated in Fig. 2a. A height $a_n$ of the nth peak, given by Eq. (11b), depends on the power amplitudes $B_1$, $B_2$ received from the chest 26 and the abdominal region 28, respectively, on the chest and abdominal region displacement amplitudes $x_0^1$ and $x_0^2$, on the difference $\Delta R = R_1 - R_2$ in the average distances from chest 26 and abdominal region 28 to the radar sensor system 10, and on the phase lag $\phi$.

It is obvious from Eq. (11b) that in this specific embodiment of the radar sensor system 10, in which a predetermined displacement along the predetermined direction relative to the subject 24 is enabled, the maximum displacement of the mechanism of 10.0 mm is sufficient to make the height $a_n$ of the nth peak undergo a full oscillation.

The predetermined direction for the predetermined displacement is chosen such that a displacement results in a change of the equilibrium distances $R_1$ and $R_2$ of the chest 26 and the abdominal region 28, respectively, as well as in a change of the quantity $\Delta R = R_1 - R_2$. In general, the power amplitudes also scale like $B_1 \sim \dfrac{1}{R_1^2}$ , but if small displacements of the radar sensor system 10 are implemented in the order of magnitude of the radar carrier wavelength $\lambda \ll R_i$, $i = 1, 2$, the amplitude change will be negligible, while the cosine function term in Eq. (11b) can vary substantially.

Values of a plurality of peaks of the calculated Fourier transform $A(\nu)$ are determined and recorded by the control and evaluation unit 22 in another step 142 of the method.

In an alternative embodiment of the method, values of a plurality of four additional peaks of the calculated Fourier transform $A(\nu)$ that represent higher order harmonics (n = 9 to 12) with respect to the plurality of peaks (n = 1 to 7) of the calculated Fourier transform $A(\nu)$ can be determined and recorded. A smallest gap between the plurality of peaks and the plurality of four additional peaks of the calculated Fourier transform is equivalent to two orders. A standstill of at least one out of a chest breathing amplitude and an abdominal region breathing amplitude can be detected by comparing the recorded values of the plurality of four additional peaks with predetermined threshold values for the plurality of four additional peaks, and by detecting that the recorded values of the plurality of four additional peaks exceed the predetermined threshold values for a predetermined period of time. The smaller the breathing amplitude, the less pronounced are any higher order frequency harmonics in the Fourier transform of the signal autocorrelation function. As an example, for a child breathing with amplitude of 2 to 3 mm, there will be only one or two higher order harmonics visible in the Fourier transform of the signal autocorrelation function for a 24 GHz radar carrier frequency. During an apnea event, 5 to 6 additional peaks will be emerging (Fig. 2b), an observation which can be exploited as an alarm trigger, as at least one of the higher order peaks may exceed the predetermined threshold value.

Referring again to the first embodiment of the method, in another step 144, the number of radar wavelengths in the path between the radar transmitting antenna 12, the subject 24 and the radar receiving antenna 16 is changed by a predetermined amount by carrying out a predetermined displacement of the radar sensor system 10 relative to the subject 24 along the predetermined direction. The steps 134 to 144 are iteratively carried out until two extreme values, namely a maximum and a minimum value, are detected for the determined values of each one the plurality of peaks.

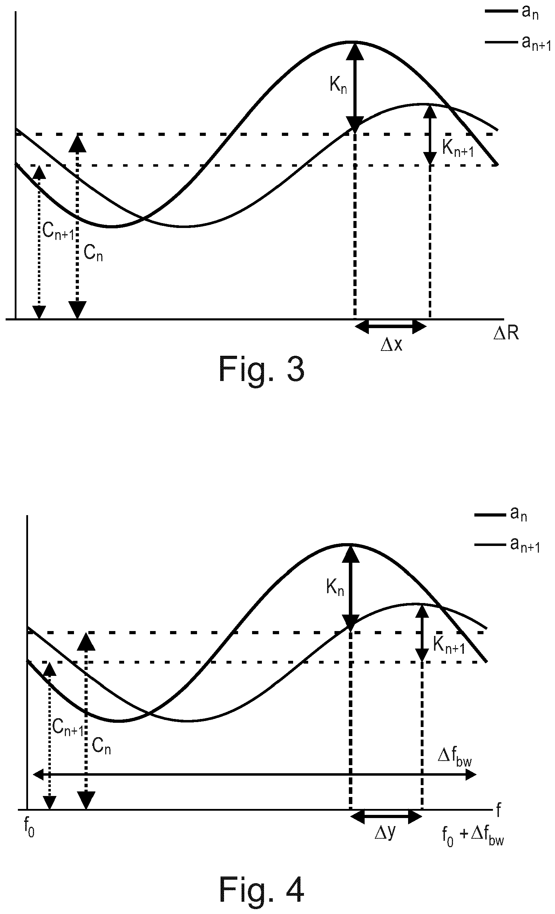

Fig. 3 exemplarily shows a plot of the variation of the recorded values of two adjacent peaks of the calculated Fourier transform $A(\nu)$ pursuant to Fig. 2a, namely a value of the nth peak $a_n$ and a value $a_{n+1}$ of the (n+1)th peak, as a result of a series of predetermined displacements of the radar sensor system 10 pursuant to Fig. 1.

From the plot of Fig. 3, a phase lag $\phi$ between the breathing motions of the chest 26 and the abdominal region 28 of the subject 24 is derived in another step 146. The phase lag $\phi$ can be determined by looking at the distance between the maxima of the nth and (n+1)th peak in Fig. 3. The phase lag $\phi$ is given by

$$\phi = 2k\pi - \frac{2\pi}{\lambda}\Delta x,$$

wherein $\Delta x$ is the distance between the maximum of the nth peak and the maximum of the (n+1)th peak, and $k$ is an integer that is chosen to fulfil $0 \le \phi \le \pi$.

The constants $C_n$ and $K_n$ are also extracted from the plot of amplitudes of two peaks of the calculated Fourier transform in another step 148 of the method. Constant $C_n$ represents the constant offset in Fig. 3, while constant $K_n$ represents the amplitude of the oscillation. This is carried out for a plurality of different values for n, e.g. n = 1, 2, 3 ... for reduced error of measurement. The constants $C_n$ and $K_n$ provide information about amplitudes of the displacements of the chest 26 and abdominal region 28 of the subject 24. It should be emphasized at this point that the constants $C_n$ and $K_n$ depend on quantities such as a particular shape of the breathing displacement (e.g. sinusoidal, triangular, etc.), breathing amplitudes, radar cross sections of chest and the abdomen, and an antenna gain pattern. However, for determining the degree of chest/abdomen asynchronicity none of these quantities (radar cross sections, antenna gain pattern, etc.) need to be known a *priori*. The constants $C_n$ and $K_n$ can be extracted from the experimentally measured variation, such as the offset and the size of the oscillation, respectively.

As optional steps of the method (not shown in Fig. 7), the determined phase lag $\phi$, chest breathing amplitude and abdominal region breathing amplitude can be compared with predetermined threshold values for each one of these determined quantities. Also, an output signal representing a breathing status of the subject 24 can be generated and provided to an external unit for further signal processing.

All predetermined, predefined, calculated or recorded values and threshold values mentioned herein reside in the digital data memory unit of the evaluation and control unit 22 and can readily be retrieved by the processor unit of the evaluation and control unit 22.

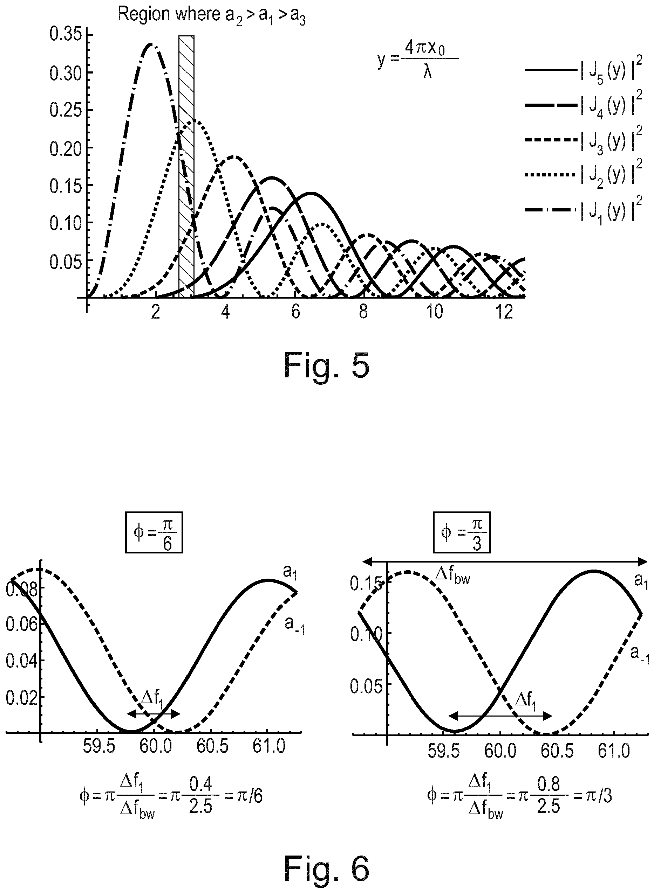

In an alternative embodiment of the method, the step of determining and recording values of at least two peaks of the calculated Fourier transform $A(\nu)$ includes determining and recording values of a peak of order n and values of a peak of order -n of the calculated Fourier transform $A(\nu)$. Fig. 6 shows a plot of the variation of values of the peak of order n and values of the peak of order -n of the calculated Fourier transform $A(\nu)$ of the autocorrelation function for two distinct values of a phase lag $\phi$.

By carrying out the Fourier transform $A(\nu)$ of the autocorrelation function, both the negative and the positive frequency spectrum is obtained, which, depending on the signal shape and the phase lag $\phi$, do not necessarily have to be mirror images of one another. From Eq. (11b) and since $J_n = J_{-n}$, it follows that $C_n = C_{-n}$, and $K_n = K_{-n}$, and further that

$$a_{-n}(f) = C_n(f) + K_n(f) \cos\left(-n\phi + \frac{4\pi}{c}f(R_1 - R_2)\right) \qquad \text{Eq. (11c)}$$

From Eq. (11b) and Eq. (11c) it is obvious that minima of $a_n(f)$ and $a_{-n}(f)$ will not occur for the same value of the carrier frequency $f$ unless the phase lag $\phi$ equals zero.

The step of determining a phase lag $\phi$ between breathing motions of the chest 26 and the abdominal region 28 of the subject 24 therefore includes determining the phase lag $\phi$ by determining a difference $\overline{\Delta f_n}$ of the radar carrier frequency $f$ at which extreme values of the peak of order n and the peak of order -n occur. The phase lag $\phi$ can then be determined from

$$\phi = \pi \frac{\overline{\Delta f_n}}{\Delta f_{bw}} + \frac{m\pi}{n} \qquad \text{Eq. (12)}$$

Herein, m is an integer which should be chosen such that the phase lag $\phi$ lies between 0 and $\pi$, and $\Delta f_{bw}$ denotes the radar carrier frequency sweep that is necessary to drive the peaks $a_n(f)$ and $a_{-n}(f)$ of the Fourier transform $A(\nu)$ through an entire oscillation.

Fig. 6 shows a plot of simulation results of a variation of values of a peak of order 1 and values of a peak of order -1 of the calculated Fourier transform of an autocorrelation function of other received radar waves for two distinct values of a phase lag $\phi$, namely for $\phi = \frac{\pi}{6}$ (left hand side) and $\phi = \frac{\pi}{3}$ (right hand side), respectively, with same other parameters. The value of the phase lag $\phi$ as determined by the above-described method was found to exactly match the phase lag $\phi$ that was entered in the simulations.

This method of determining the phase lag $\phi$ from the features of positive and negative Fourier spectra is especially useful in the case of breathing amplitudes that are small compared to the radar carrier wavelength, when only the peaks of order 1 and -1 are visible in the Fourier spectrum, whereas any higher order peaks with n = $\pm 2$, $\pm 3$, ... might not exist.

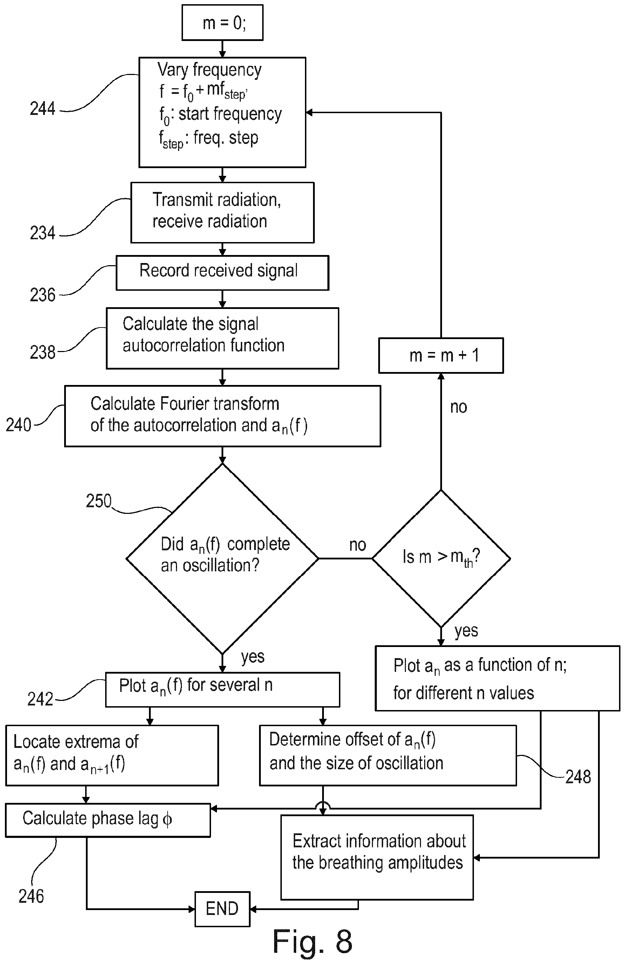

Fig. 8 shows a flowchart of an alternative embodiment of the method in accordance with the invention, of operating the radar sensor system 10 pursuant to Fig. 1 for monitoring a breathing motion of the subject 24.

With the radar sensor system 10 arranged at a fixed position relative to the subject 24, the radar transmitting unit is controlled by the control and evaluation unit 22 for transmitting radar waves of a predetermined initial radar carrier frequency $f_0$ for a specified duration towards the chest 26 and the abdominal region 28 of the subject 24 in a step 234 of the method.

The radar receiving unit receives radar waves that have been transmitted by the radar transmitting unit and that have been reflected by the chest 26 and the abdominal region 28 of the subject 24, for example for a duration of 10 s, and the radar receiving unit generates received radar signals from the received radar waves. In the next step 236, the generated received radar signal is received and recorded by the control and evaluation unit 22.

In another step 238 of the method, the autocorrelation function of the received radar signal is calculated. In a next step 240, the Fourier transform or a similar transformation of the calculated autocorrelation function is calculated.

Values of a plurality of peaks of the calculated Fourier transform are determined and recorded by the control an evaluation unit 22 in another step 242 of the method.

In another step 244, the number of radar wavelengths in the path between the radar transmitting antenna 12, the subject 24 and the radar receiving antenna 16 is changed by a predetermined amount by changing the radar carrier frequency of the radar sensor system by a predetermined frequency amount. The steps 234 to 244 are iteratively carried out until two extreme values, namely a maximum and a minimum value, are detected for the determined values of each one the plurality of peaks.

Fig. 4 exemplarily shows a plot of the variation of the recorded values of two adjacent peaks of the calculated Fourier transform $A(\nu)$ pursuant to Fig. 2a, namely a value of the nth order peak $a_n$ and a value $a_{n+1}$ of the (n+1)th order peak, as a result of predetermined changes of the radar carrier frequency of the radar sensor system 10 pursuant to Fig. 1. The predetermined changes of the radar carrier frequency are performed by tuning a control voltage at a control input line of a voltage controlled oscillator (VCO, not shown) of the radar transmitting unit, as is commonly known in the art.

From the plot of Fig. 4, a phase lag $\phi$ between the breathing motion of the chest 26 and the abdominal region 28 of the subject 24 is derived in another step 246. The phase lag $\phi$ can be determined from an observed difference $\Delta y$ of the radar carrier frequencies at which the maxima of the nth and (n+1)th order peak are located. The phase lag $\phi$ is then given by

$$\phi = 2\pi \frac{\Delta y}{\Delta f_{bw}},$$

wherein $\Delta f_{bw}$ corresponds to the frequency sweep bandwidth that is necessary to observe a full oscillation of the term $a_n$.

The constants $C_n$ and $K_n$ are also extracted from the plot of amplitudes of two peaks of the calculated Fourier transform $A(\nu)$ in another step 248 of the method. Constant $C_n$ represents the constant offset in Fig. 4, while constant $K_n$ represents the amplitude of the oscillation. This is carried out for a plurality of different values for n, e.g. n = 1, 2, 3 ... for reduced error of measurement. The constants $C_n$ and $K_n$ provide information about amplitudes of the chest and abdominal region displacement.

The behavior of the determined and recorded values of the plurality of peaks of the calculated Fourier transform $A(\nu)$ is checked for periodicity in another step 250 as the carrier frequency is varied. If it is observed that the determined and recorded values of the plurality of peaks of the calculated Fourier transform $A(\nu)$ do not show a periodic behavior, then the configuration of the radar sensor system 10 and the subject 24 is such that $R_1 - R_2 \ll \lambda$. From Eq. (11b) one can obtain that the second term in the argument of the cosine function becomes negligible, which is more apparent if the term is rephrased, using the relation $\frac{1}{\lambda} = \frac{f}{c}$ for electromagnetic waves, to $4\pi \frac{R_1 - R_2}{\lambda}$ .

Thus, $a_n$ can be approximated by

$$a_n(f) \approx C_n(\bar{f}) + K_n(\bar{f})\cos(n\phi), \qquad \text{Eq. (12)}$$

wherein $\bar{f} = f_0 + \Delta f_{bw}/2$ is the mean value of the radar carrier frequency during the frequency sweep. In this case, the constants $C_n$ and $K_n$ and, by that, amplitudes of the chest and abdominal region displacements can directly be determined from Eq. (12), using the values of $a_n$ for different values of n.

In the flowchart shown in Fig. 8, $m_{th}$ denotes a predetermined threshold value for a maximum number of stepwise increases of the radar carrier frequency, that is necessary for $a_n$ to undergo at least one full oscillation.

In another alternative embodiment of the method, values of at least three peaks of a calculated Fourier transform of an autocorrelation function of other received radar waves are determined and recorded. Fig. 5 shows a plot of values of the first five peaks of consecutive order of another calculated Fourier transform of an autocorrelation function of other received radar waves vs. a breathing amplitude (i.e. parameter $y = \frac{4\pi x_0}{\lambda}$ ) for a fixed radar carrier frequency. A chest breathing amplitude or an abdominal region breathing amplitude can be estimated by carrying out a ranking of the determined values of the peaks of the first three orders.

Considering a sinusoidal breathing pattern for demonstration purposes, the value $a_n$ of the nth order peak (i.e. the peak located at $nf_b$, wherein n is an integer and $f_b$ the breathing frequency) is proportional to $J_n^2(4\pi \frac{x_0}{\lambda})$ , with $x_0$ denoting the breathing displacement amplitude, $\lambda$ the wavelength and $J_n$ the nth order Bessel function of the first kind (for a sinusoidal breathing pattern). If, for a given wavelength and breathing amplitude, there are multiple peaks present in the Fourier transform of the autocorrelation function, one can carry out a ranking of the peaks according to their value.

For example $a_2 > a_1 > a_3$, means that the value of peak of order $n = 2$ is the largest, followed by the value of the peak of order $n = 1$, which is the second largest, and then the third largest value is that of the peak of order n = 3. To verify this ranking in the plot of Fig. 5, the conditions of $J_2^2\left(4\pi\frac{x_0}{\lambda}\right) > J_1^2\left(4\pi\frac{x_0}{\lambda}\right) > J_3^2\left(4\pi\frac{x_0}{\lambda}\right)$ have to be met, and this only holds for the predetermined interval $2.5 < 4\pi\frac{x_0}{\lambda} < 3.05$ (other potential intervals are excluded by plausibility considerations). Thus, an estimate of the breathing displacement amplitude can be determined by identifying one out of a plurality of predetermined breathing amplitude intervals assigned to rankings of the peaks of the first three orders of the Fourier transform of the autocorrelation function.

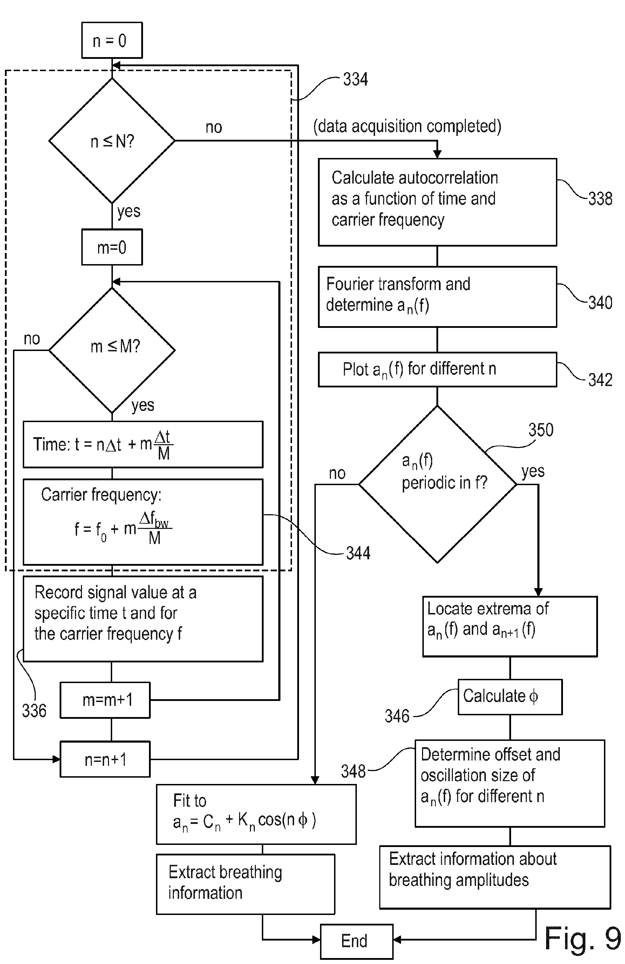

Fig. 9 shows a flowchart of another alternative embodiment of a method in accordance with the invention of operating the radar sensor system 10 pursuant to Fig. 1 for monitoring a breathing motion of the subject 24.

In this embodiment of the method, the step 334 of operating the radar transmitting unit for transmitting radar waves includes transmitting a plurality of N+1 consecutive radar wave trains 30 as illustrated in Fig. 8. Each wave train 30 has a duration $\Delta t$ and comprises a plurality of M+1 radar waves 32 of predetermined equal duration $\frac{\Delta t}{M+1}$, wherein radar carrier frequencies $f$ of each radar wave 32 of the plurality of M+1 radar waves 32 differ by an integral multiple of the predetermined frequency amount $\frac{\Delta f_{bw}}{M}$.

Time $t(m, n)$ and radar carrier frequency $f(m)$ of the mth radar wave 32 of the nth radar wave train 30 are given by

$$t(m, n) = n\Delta t + m\frac{\Delta t}{M}, m = 0,1,\dots,M; n = 0, 1, \dots, N$$

$$f(m) = f_o + m\frac{\Delta f_{bw}}{M}, m = 0, 1, \dots, M, \qquad\qquad \text{Eq. (13)}$$

wherein $f_0$ denotes an initial radar carrier frequency. Thus, the step 334 of operating the radar transmitting unit includes a step 344 of changing the number of radar wavelengths in the path between the radar transmitting antenna 12, the subject 24 and the radar receiving antenna 16 by predetermined amounts by changing the radar carrier frequency of the radar sensor system. In the exemplary embodiment shown in Fig. 8, M equals 5.

Further, a step 336 of operating the radar receiving unit for receiving radar waves includes receiving radar waves 18 that have been reflected by the chest 26 and abdominal region 28 of the subject 24 for each radar wave 32 of the plurality of radar waves 32 and for each radar wave train 30 of the plurality of radar wave trains 30, i.e. at each point of time $t(m,n)$ as given in Eq. (13), and includes generating and recording received radar signals for each one of the received radar waves 32.

After the plurality of N+1 consecutive radar wave trains 30 has been transmitted and the radar signals recorded, which takes a measurement time $T_{rec}$ of

$$T_{rec} = (N + 1)\Delta t,$$

the autocorrelation function of the received radar signal is calculated in another step 338 of the method as a function of time $t$ and radar carrier frequency $f$.

While in the embodiment of the method pursuant to Fig. 8, the radar signal needs to be recorded for every radar carrier frequency $f \in (f_0, f_0 + \Delta f_{bw})$ for a duration of e.g. 10 s, and thus may require a somewhat longer total measurement time, which is equal to the number of possible radar carrier frequencies times 10 s, in the embodiment of the method pursuant to Fig. 9, the total measurement time can be reduced to $T_{rec} \approx 10$ s, and, nevertheless, the radar signal values at different values of the radar carrier frequency $f \in (f_0, f_0 + \Delta f_{tbw})$ and for the times $t(m, n) \in (0, T_{rec})$ have been recorded. In order to accomplish this shorter measurement time, the radar carrier frequency sweep shall be suitably and sufficiently fast in this embodiment.

In a next step 340, the Fourier transform or a similar transformation of the calculated autocorrelation function is calculated. A plurality of peaks of the calculated Fourier transform $A(v)$ is determined and recorded in another step 342.

In another step 350, the periodicity of the determined and recorded values of the plurality of peaks of the calculated Fourier transform $A(v)$ is checked either during a carrier frequency sweep or during radar displacement. If a periodic behavior is detected, a phase lag $\phi$ between the breathing motion of the chest 26 and the abdominal region 28 of the subject 24 is derived in another step 346 as described for the embodiment pursuant to Fig. 6. Also, constants $C_n$ and $K_n$ are extracted from the plot of amplitudes of two peaks of the calculated Fourier transform $A(v)$ in another step 348 of the method as described for the embodiment pursuant to Fig. 8.

If it is observed that the determined and recorded values of the plurality of peaks of the calculated Fourier transform $A(v)$ do not show a periodic behavior, the constants $C_n$ and $K_n$ and, by that, amplitudes of the chest and abdominal region displacements can directly be determined from Eq. (12), using the values of $a_n$ for different values of n.

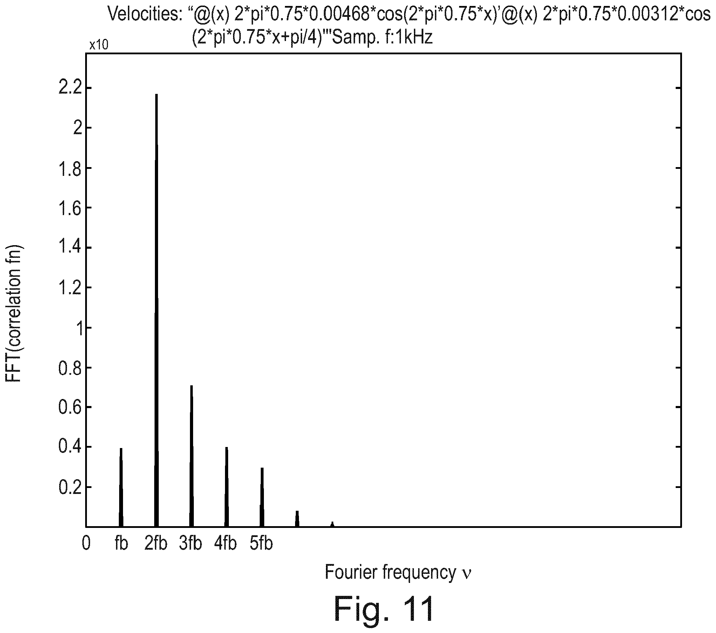

In yet another possible embodiment the carrier frequency does not need to be changed and the chest and abdomen displacement amplitudes can be predetermined (e.g. by pulse radar, ultrasound etc.). In this specific embodiment the phase lag between the chest and abdominal motion is then extracted by comparing the pattern of the Fourier transform of the autocorrelation function to the library of patterns for different values of phase lag, until the best match is found, see for example illustrated in Fig. 11.

Fig. 11 shows the simulation of the Fourier transform of the autocorrelation function for the chest-abdomen phase lag of $\phi = \pi/4$ and unequal chest and abdomen amplitudes. Herein, $f_b$ denotes the breathing frequency. We have taken equal chest and abdomen equilibrium distances $R_1 = R_2$, equal chest and abdomen radar cross sections and

$$x_0^1 = 4.68mm \text{ and } x_0^2 = 3.12mm$$

for the displacement amplitudes. The carrier frequency was chosen to be 24 GHz. As expected from theory, see Eq. (11b), the ratio of peaks is $\frac{a_2}{a_1} = 5.45$, and $\frac{a_3}{a_1} = 1.79$, $\frac{a_4}{a_1} = 1.02$, and

$$\frac{a_5}{a_1} = 0.778$$

. There is an excellent agreement between the analytics and simulations.

While the invention has been illustrated and described in detail in the drawings and foregoing description, such illustration and description are to be considered illustrative or exemplary and not restrictive; the invention is not limited to the disclosed embodiments but it is defined by appended claims 1-14.

### List of Reference Symbols

| | | | |
|---|---|---|---|
| 10 | radar sensor system | 22 | evaluation and control unit |
| 12 | radar transmitting antenna | 24 | subject |
| 14 | transmitted radar waves | 26 | chest |
| 16 | radar receiving antenna | 28 | abdominal region |
| 18 | reflected radar waves | 30 | wave train |
| 20 | transceiver unit | 32 | radar wave |

Steps of

| | |
|---|---|
| 34 | transmit radar waves |
| 36 | receive radar waves and record radar signals |
| 38 | calculate autocorrelation function of received radar signal |
| 40 | calculate Fourier transform of autocorrelation function |
| 42 | record values of peaks of calculated Fourier transform |
| 44 | change number of radar wavelengths in path to subject |
| 46 | derive phase lag |
| 48 | extract breathing amplitudes |
| 50 | check for periodicity |
| A(v) | Fourier transform of autocorrelation function |
| $v$ | Fourier frequency component |
| $a_n$ | height of nth peak of Fourier transform A(v) |
| $C_n$ | constant offset |
| $\overline{\Delta f_n}$ | radar carrier frequency difference between extrema of $a_n$ and $a_{-n}$ |
| $(f_0), f$ | (initial) radar carrier frequency |
| $f_b$ | chest and abdominal region motion frequency |
| $\Delta f_{bw}$ | frequency sweep bandwidth for full oscillation |
| $\phi$ | phase lag between breathing motions of chest and abdominal region of subject |
| $K_n$ | amplitude of oscillation |

ΔR      difference of average distances between chest and abdominal region to radar sensor system

Δx      distance between maximum of nth peak and (n+1)th peak

Δy      difference Δy of radar carrier frequencies between maximum of nth peak and (n+1)th peak

**Claims**

1.  A method of operating a radar sensor system (10) for monitoring a breathing motion of a subject (24), the radar sensor system (10) including a radar transmitting unit having one radar transmitting antenna (12) and being configured for transmitting radar waves (14) towards the subject (24), a radar receiving unit having one radar receiving antenna (16) and being configured for receiving radar waves (18) that have been transmitted by the radar transmitter unit and have been reflected by the subject (24), and an evaluation and control unit (22) that is at least configured for evaluating Doppler information from the radar waves (18) received by the radar receiving unit,
    the method comprising at least steps of

    - operate (34) the radar transmitting unit for at least once transmitting radar waves (14) of a predetermined radar carrier frequency ($f$) for a specified time towards a chest (26) and an abdominal region (28) of the subject (24),
    - operate (36) the radar receiving unit at least once for a specified time for receiving radar waves (18) that have been transmitted by the radar transmitting unit and that have been reflected by the chest (26) and the abdominal region (28) of the subject (24), and for generating received radar signals from the received radar waves (18),
    - calculate (38) the autocorrelation function of the received radar signals,
    - calculate (40) the Fourier transform $A(\nu)$ of the calculated autocorrelation function,
    - determine and record (42) values of at least two peaks of the calculated Fourier transform $A(\nu)$,
    - change (44) the number of radar wavelengths in the path between the radar transmitting antenna (12), the subject (24) and the radar receiving antenna (16) at least once by a predetermined amount,
    - iteratively carry out the preceding steps (34 to 44) until at least one extreme value is detected for the determined values of each one the at least two peaks, and
    - from the recorded determined and detected extreme values of the at least two peaks, determine (46, 48) at least one breathing-characteristic parameter of the breathing motion of the subject (24).

2.  The method as claimed in claim 1, wherein the step (44) of changing the number of radar wavelengths in the path includes carrying out a predetermined displacement along a predetermined direction of at least one out of the radar transmitting antenna (12) and the radar receiving antenna (16) relative to the subject (24).

3.  The method as claimed in any one of the preceding claims 1 or 2, wherein the step (44) of changing the number of radar wavelengths in the path includes changing a radar carrier frequency ($f_0$, $f$) of the radar sensor system (10) by a predetermined amount.

4.  The method as claimed in claim 3, wherein

    - the step (34) of operating the radar transmitting unit for transmitting radar waves (14) includes transmitting a plurality of consecutive radar wave trains (30), each wave train (30) comprising a plurality of radar waves (32) of predetermined duration, wherein radar carrier frequencies ($f_0$, $f$) of each radar wave (32) of the plurality of radar waves (32) differ by an integral multiple of the predetermined amount, and
    - the step (36) of operating the radar receiving unit for receiving radar waves includes receiving radar waves (18) that have been reflected by the chest (26) and abdominal region (28) of the subject (24) for each radar wave (32) of the plurality of radar waves (32) and for each radar wave train (30) of the plurality of radar wave trains (30), and includes generating and recording received radar signals for each one of the received radar waves (32).

5.  The method as claimed in any one of the preceding claims, wherein the step (44) of changing the number of radar wavelengths in the path is carried out until at least one minimum value and one maximum value is recorded for the determined values of each one of the at least two peaks of the calculated Fourier transform $A(\nu)$ of the autocorrelation function $A(\tau)$.

6.  The method as claimed in any one of the preceding claims, wherein the step (48) of determining at least one breathing-characteristic parameter of the breathing motion of the subject (24) includes correcting the at least one extreme value for the determined values of one of the at least two peaks by an offset value determined from the recorded determined values of the one of the at least two peaks and/or evaluating information contained in said offset value.

7. The method as claimed in any one claims 1 to 5, wherein the step of determining and recording (42) values of at least two peaks of the calculated Fourier transform $A(\nu)$ includes determining and recording values of a peak of order n and values of a peak of order -n of the calculated Fourier transform $A(\nu)$, and wherein the step of determining at least one breathing-characteristic parameter of the breathing motion of the subject (24) includes determining a phase lag ($\phi$) between breathing motions of the chest and the abdominal region of the subject by determining a difference of the radar carrier frequencies at which extreme values of the peak of order n and the peak of order -n occur.

8. The method as claimed in claim 1, wherein the step of determining and recording (42) values of at least two peaks of the calculated Fourier transform $A(\nu)$ comprises determining and recording a plurality of at least two additional peaks of the calculated Fourier transform $A(\nu)$ representing higher order harmonics with respect to the at least two peaks, wherein the step of determining (46, 48) at least one breathing-characteristic parameter of the breathing motion of the subject (24) comprises determining at least one out of determining a chest breathing amplitude and an abdominal region breathing amplitude, and further comprises determining a standstill of the at least one out of a chest breathing amplitude and an abdominal region breathing amplitude by comparing recorded values of the plurality of at least two additional peaks of the calculated Fourier transform $A(\nu)$ with predetermined threshold values for the plurality of at least two additional peaks of the calculated Fourier transform $A(\nu)$, and by detecting that recorded values of the plurality of at least two additional peaks of the calculated Fourier transform $A(\nu)$ exceed the predetermined threshold values for a predetermined period of time.

9. The method as claimed in claim 1, wherein the step of determining and recording (42) comprises determining and recording values of at least three peaks of the calculated Fourier transform $A(\nu)$, and wherein the step of determining (46, 48) at least one breathing-characteristic parameter of the breathing motion of the subject (24) includes determining one out of a plurality of predetermined breathing amplitude intervals based on carrying out a ranking of the determined values of the at least three peaks.

10. The method as claimed in claim 7, further comprising steps of

- comparing the determined at least one out of phase lag ($\phi$), chest breathing amplitude and abdominal region breathing amplitude with at least one predetermined threshold value, and
- generating an output signal representing a breathing status of the subject (24).

11. A radar sensor system (10) for monitoring a breathing motion of a subject (24), the radar sensor system (10) comprising

- a radar transmitting unit having one radar transmitting antenna (12) and being configured for transmitting radar waves (14) of a predetermined radar carrier frequency ($f_0, f$) towards a chest (26) and an abdominal region (28) of the subject (24),
- a radar receiving unit having one radar receiving antenna (16) and being configured for receiving radar waves (18) that have been transmitted by the radar transmitter unit and have been reflected by the subject (24), and
- an evaluation and control unit (22) that is at least configured for evaluating Doppler information from the radar waves (18) received by the radar receiving unit, and that is further configured to automatically execute steps of the method as claimed in any one of the preceding claims.

12. The radar sensor system (10) as claimed in claim 11 wherein the predetermined radar carrier frequency ($f_0, f$) is lying in a frequency range between 15 GHz and 130 GHz and more preferably in a frequency range between 57 GHz and 64 GHz.

13. The radar sensor system (10) as claimed in claims 11 or 12, wherein the radar transmitting antenna (12) is configured to have a main lobe that is able to cover a major part of the chest (26) and the abdominal region (28) of the subject (24).

14. A software module for controlling automatic execution of the method as claimed in any one of claims 1 to 10, wherein method steps to be conducted are converted into a program code of the software module, wherein the program code is implementable in a digital data memory unit of the radar sensor system or a separate control unit and is executable by a processor unit of the radar sensor system or a separate control unit, interfaced with the radar sensor system.

**Patentansprüche**

1. Verfahren zum Betreiben eines Radarsensorsystems (10) zur Überwachung einer Atmungsbewegung eines Subjekts (24), wobei das Radarsensorsystem (10) eine Radarsendeeinheit mit einer Radarsendeantenne (12), die dafür ausgelegt ist, Radarwellen (14) zu dem Subjekt (24) zu übertragen, eine Radarempfangseinheit mit einer Radarempfangsantenne (16), die dafür ausgelegt ist, Radarwellen (18) zu empfangen, die von der Radarsendereinheit übertragen und von dem Subjekt (24) reflektiert worden sind, und eine Auswerte- und Steuereinheit (22) aufweist, die zumindest zum Auswerten von Dopplerinformationen von den von der Radarempfangseinheit empfangenen Radarwellen (18) eingerichtet ist, wobei das Verfahren mindestens die folgenden Schritte aufweist:

   - Betreiben (34) der Radarsendeeinheit, um mindestens einmal Radarwellen (14) mit einer vorbestimmten Radarträgerfrequenz (f) während einer spezifizierten Zeit zu einer Brust (26) und einem Bauchbereich (28) des Subjekts (24) zu übertragen,
   - Betreiben (36) der Radarempfangseinheit mindestens einmal während einer spezifizierten Zeit, um Radarwellen (18) zu empfangen, die von der Radarsendeeinheit übertragen und von der Brust (26) und dem Bauchbereich (28) des Subjekts (24) reflektiert worden sind, und um aus den empfangenen Radarwellen (18) empfangene Radarsignale zu erzeugen,
   - Berechnen (38) der Autokorrelationsfunktion der empfangenen Radarsignale,
   - Berechnen (40) der Fouriertransformation $A(v)$ der berechneten Autokorrelationsfunktion,
   - Bestimmen und Aufzeichnen (42) von Werten von mindestens zwei Spitzenwerten der berechneten Fouriertransformation $A(v)$,
   - Ändern (44) der Anzahl an Radarwellenlängen in dem Weg zwischen der Radarsendeantenne (12), dem Subjekt (24) und der Radarempfangsantenne (16) mindestens einmal um einen vorbestimmten Betrag,
   - wiederholtes Ausführen der vorausgehenden Schritte (34 bis 44), bis mindestens ein Extremwert für die bestimmten Werte von jedem der mindestens zwei Spitzenwerte erkannt ist, und
   - anhand der aufgezeichneten bestimmten und erkannten Extremwerte der mindestens zwei Spitzenwerte Bestimmen (46, 48) mindestens eines Atmungscharakteristik-Parameters der Atmungsbewegung des Subjekts (24).

2. Verfahren nach Anspruch 1, wobei der Schritt (44) des Änderns der Anzahl an Radarwellenlängen in dem Weg das Ausführen einer vorbestimmten Verschiebung entlang einer vorbestimmten Richtung von mindestens einer von der Radarsendeantenne (12) und der Radarempfangsantenne (16) relativ zum Subjekt (24) umfasst.

3. Verfahren nach einem der vorhergehenden Ansprüche 1 oder 2, wobei der Schritt (44) des Änderns der Anzahl an Radarwellenlängen in dem Weg das Ändern einer Radarträgerfrequenz ($f_0$, $f$) des Radarsensorsystems (10) um einen vorbestimmten Betrag umfasst.

4. Verfahren nach Anspruch 3, wobei

   - der Schritt (34) des Betreibens der Radarsendeeinheit zum Übertragen von Radarwellen (14) das Übertragen von mehreren aufeinander folgenden Radarwellenzügen (30) umfasst, wobei jeder Wellenzug (30) mehrere Radarwellen (32) einer vorbestimmten Dauer aufweist, wobei sich Radarträgerfrequenzen ($f_0$, $f$) jeder Radarwelle (32) der mehreren Radarwellen (32) um ein ganzzahliges Vielfaches von dem vorbestimmten Betrag unterscheiden, und
   - der Schritt (36) des Betreibens der Radarempfangseinheit zum Empfangen von Radarwellen das Empfangen von Radarwellen (18), die von der Brust (26) und dem Bauchbereich (28) des Subjekts (24) für jede Radarwelle (32) der mehreren Radarwellen (32) und für jeden Radarwellenzug (30) der mehreren Radarwellenzüge (30) reflektiert worden sind, und das Erzeugen und Aufzeichnen von empfangenen Radarsignalen für jede der empfangenen Radarwellen (32) umfasst.

5. Verfahren nach einem der vorhergehenden Ansprüche, wobei der Schritt (44) des Änderns der Anzahl an Radarwellenlängen in dem Weg ausgeführt wird, bis mindestens ein Mindestwert und ein Höchstwert für die bestimmten Werte von jedem der mindestens zwei Spitzenwerte der berechneten Fouriertransformation $A(v)$ der Autokorrelationsfunktion $A(\tau)$ aufgezeichnet wird.

**6.** Verfahren nach einem der vorhergehenden Ansprüche, wobei der Schritt (48) des Bestimmens mindestens eines Atmungscharakteristik-Parameters der Atmungsbewegung des Subjekts (24) das Korrigieren des mindestens einen Extremwertes für die bestimmten Werte von einem der mindestens zwei Spitzenwerte um einen Korrekturwert umfasst, der anhand der aufgezeichneten bestimmten Werte des einen der mindestens zwei Spitzenwerte und/oder durch Auswerten von in dem Korrekturwert enthaltenen Informationen bestimmt wird.

**7.** Verfahren nach einem der Ansprüche 1 bis 5, wobei der Schritt des Bestimmens und Aufzeichnens (42) von Werten von mindestens zwei Spitzenwerten der berechneten Fouriertransformation $A(\nu)$ das Bestimmen und Aufzeichnen von Werten eines Spitzenwertes der Ordnung n und Werten eines Spitzenwertes der Ordnung -n der berechneten Fouriertransformation $A(\nu)$ aufweist, und wobei der Schritt des Bestimmens mindestens eines Atmungscharakteristik-Parameters der Atmungsbewegung des Subjekts (24) das Bestimmen einer Phasenverzögerung ($\Phi$) zwischen Atmungsbewegungen der Brust und des Bauchbereichs des Subjekts durch Bestimmen einer Differenz der Radarträgerfrequenzen, bei denen Extremwerte des Spitzenwertes der Ordnung n und des Spitzenwertes der Ordnung -n auftreten, aufweist.

**8.** Verfahren nach Anspruch 1, wobei der Schritt des Bestimmens und Aufzeichnens (42) von Werten von mindestens zwei Spitzenwerten der berechneten Fouriertransformation $A(\nu)$ das Bestimmen und Aufzeichnen von mehreren von mindestens zwei zusätzlichen Spitzenwerten der berechneten Fouriertransformation $A(\nu)$ umfasst, welche Harmonische höherer Ordnung in Bezug auf die mindestens zwei Spitzenwerte darstellen, wobei der Schritt des Bestimmens (46, 48) mindestens eines Atmungscharakteristik-Parameters der Atmungsbewegung des Subjekts (24) das Bestimmen von mindestens einem von dem Bestimmen von einer Brust-Atmungsamplitude und einer Bauchbereich-Atmungsamplitude umfasst, und ferner das Bestimmen eines Stillstands der mindestens einen von einer Brust-Atmungsamplitude und einer Bauchbereich-Atmungsamplitude umfasst, durch Vergleichen von aufgezeichneten Werten der mehreren von mindestens zwei zusätzlichen Spitzenwerten der berechneten Fouriertransformation $A(v)$ mit vorbestimmten Schwellenwerten für die mehreren von mindestens zwei zusätzlichen Spitzenwerte der berechneten Fouriertransformation $A(v)$, und durch Erkennen, dass aufgezeichnete Werte der mehreren von mindestens zwei zusätzlichen Spitzenwerten der berechneten Fouriertransformation $A(v)$ die vorbestimmten Schwellenwerte für einen vorbestimmten Zeitraum übersteigen.

**9.** Verfahren nach Anspruch 1, wobei der Schritt des Bestimmens und Aufzeichnens (42) das Bestimmen und Aufzeichnen von Werten von mindestens drei Spitzenwerten der berechneten Fouriertransformation $A(\nu)$ umfasst und wobei der Schritt des Bestimmens (46, 48) mindestens eines Atmungscharakteristik-Parameters der Atmungsbewegung des Subjekts (24) das Bestimmen von einem von mehreren vorbestimmten Atmungsamplitudenintervallen basierend auf dem Ausführen einer Rangfolge der bestimmten Werte der mindestens drei Spitzenwerte umfasst.

**10.** Verfahren nach Anspruch 7, ferner aufweisend die folgenden Schritte:

- Vergleichen des bestimmten mindestens einen Wertes von der Phasenverzögerung ($\Phi$), der Brust-Atmungsamplitude und der Bauchbereich-Atmungsamplitude mit mindestens einem vorbestimmten Schwellenwert, und
- Erzeugen eines Ausgangssignals, das einen Atmungszustand des Subjekts (24) darstellt.

**11.** Radarsensorsystem (10) zur Überwachung einer Atmungsbewegung eines Subjekts (24), wobei das Radarsensorsystem (10) aufweist:

- eine Radarsendeeinheit mit einer Radarsendeantenne (12), die dafür ausgelegt ist, Radarwellen (14) mit einer vorbestimmten Radarträgerfrequenz ($f_0$, $f$) zu einer Brust (26) und einem Bauchbereich (28) des Subjekts (24) zu übertragen,
- eine Radarempfangseinheit mit einer Radarempfangsantenne (16), die dafür ausgelegt ist, Radarwellen (18) zu empfangen, die von der Radarsendereinheit übertragen und von dem Subjekt (24) reflektiert worden sind, und
- eine Auswerte- und Steuereinheit (22), die mindestens dafür ausgelegt ist, Dopplerinformationen von den Radarwellen (18), die von der Radarempfangseinheit empfangen werden, auszuwerten, und die ferner dafür ausgelegt ist, automatisch Schritte des Verfahrens nach einem der vorhergehenden Ansprüche auszuführen.

**12.** Radarsensorsystem (10) nach Anspruch 11, wobei die vorbestimmte Radarträgerfrequenz ($f_0$, $f$) in einem Frequenzbereich zwischen 15 GHz und 130 GHz und stärker bevorzugt in einem Frequenzbereich zwischen 57 GHz und 64 GHz liegt.

**13.** Radarsensorsystem (10) nach Anspruch 11 oder 12, wobei die Radarsendeantenne (12) dafür ausgelegt ist, eine Hauptkeule aufzuweisen, die in der Lage ist, einen Großteil der Brust (26) und des Bauchbereichs (28) des Subjekts (24) abzudecken.

**14.** Softwaremodul zum Steuern einer automatischen Ausführung des Verfahrens nach einem der Ansprüche 1 bis 10, wobei auszuführende Verfahrensschritte in einen Programmcode des Softwaremoduls umgewandelt sind, wobei der Programmcode in einer digitalen Datenspeichereinheit des Radarsensorsystems oder einer separaten Steuereinheit implementierbar und von einer Prozessoreinheit des Radarsensorsystems oder einer separaten Steuereinheit, die an das Radarsensorsystem angeschlossen ist, ausführbar ist.

**Revendications**

**1.** Procédé de fonctionnement d'un système capteur radar (10) destiné à surveiller un mouvement de respiration d'un sujet (24), le système capteur radar (10) comprenant une unité de transmission radar ayant une antenne de transmission radar (12) et étant conçu pour transmettre des ondes radar (14) vers le sujet (24), une unité de réception radar ayant une antenne de réception radar (16) et étant conçue pour recevoir des ondes radar (18) qui ont été transmises par l'unité de transmission radar et qui ont été reflétées par le sujet (24), et une unité d'évaluation et de commande (22) qui est au moins conçue pour évaluer des informations Doppler provenant des ondes radar (18) reçues par l'unité de réception radar,

le procédé comprenant au moins les étapes consistant à

- faire fonctionner (34) l'unité de transmission radar pour au moins une fois transmettre des ondes radar (14) d'une fréquence porteuse radar prédéterminée ($f$) sur une durée spécifiée vers une poitrine (26) et une région abdominale (28) du sujet (24),
- faire fonctionner (36) l'unité de réception radar au moins une fois sur une durée spécifiée pour recevoir des ondes radar (18) qui ont été transmises par l'unité de transmission radar et qui ont été reflétées par la poitrine (26) et la région abdominale (28) du sujet (24), et pour générer des signaux radar reçus des ondes radar (18) reçues,
- calculer (38) la fonction d'autocorrélation des signaux radar reçus,
- calculer (40) la transformée de Fourier $A(\nu)$ de la fonction d'autocorrélation calculée,
- déterminer et enregistrer (42) des valeurs d'au moins deux pics de la transformée de Fourier $A(\nu)$ calculée,
- changer (44) le nombre de longueurs d'ondes radar dans le trajet entre l'antenne de transmission radar (12), le sujet (24) et l'antenne de réception radar (16) au moins une fois selon une quantité prédéterminée,
- exécuter de manière itérative les étapes précédentes (34 à 44) jusqu'à ce qu'au moins une valeur extrême soit détectée pour les valeurs déterminées de chacun des au moins deux pics, et
- à partir des valeurs extrêmes déterminées et détectées enregistrées des au moins deux pics, déterminer (46, 48) au moins un paramètre de caractéristique de respiration du mouvement de respiration du sujet (24).

**2.** Procédé tel que revendiqué selon la revendication 1, l'étape (44) de changement du nombre de longueurs d'ondes radar dans le trajet comprenant l'exécution d'un déplacement prédéterminé le long d'un sens prédéterminé d'au moins l'une parmi l'antenne de transmission radar (12) et l'antenne de réception radar (16) par rapport au sujet (24).

**3.** Procédé tel que revendiqué selon l'une quelconque des revendications précédentes 1 ou 2, l'étape (44) de changement du nombre de longueurs d'ondes radar dans le trajet comprenant le changement d'une fréquence porteuse radar ($f_0$, $f$) du système capteur radar (10) selon une quantité prédéterminée.

**4.** Procédé tel que revendiqué selon la revendication 3,

- l'étape (34) de fonctionnement de l'unité de transmission radar pour transmettre des ondes radar (14) comprenant la transmission d'une pluralité de trains d'ondes radar (30) consécutifs, chaque train d'ondes (30) comprenant une pluralité d'ondes radar (32) de durée prédéterminée, les fréquences porteuses radar ($f_0$, $f$) de chaque onde radar (32) de la pluralité des ondes radar (32) différant d'un multiple entier de la quantité prédéterminée, et
- l'étape (36) de fonctionnement de l'unité de réception radar pour recevoir des ondes radar comprenant la réception d'ondes radar (18) qui ont été reflétées par la poitrine (26) et la région abdominale (28) du sujet (24) pour chaque onde radar (32) de la pluralité d'ondes radar (32) et pour chaque train d'ondes radar (30) de la pluralité de trains d'ondes radar (30), et comprenant la génération et l'enregistrement de signaux radar reçus pour chacune des ondes radar (32) reçues.

5. Procédé tel que revendiqué selon l'une quelconque des revendications précédentes, l'étape (44) de changement du nombre de longueurs d'ondes radar dans le trajet étant exécutée jusqu'à ce qu'au moins une valeur minimale et une valeur maximale sont enregistrées pour les valeurs déterminées de chacun des au moins deux pics de la transformée de Fourier $A(\nu)$ calculée de la fonction d'autocorrélation $A(\tau)$.

6. Procédé tel que revendiqué selon l'une quelconque des revendications précédentes, l'étape (48) de détermination d'au moins un paramètre de caractéristique de respiration du mouvement de respiration du sujet (24) comprenant la correction de la au moins une valeur extrême pour les valeurs déterminées de l'un des au moins deux pics d'une valeur de compensation déterminée à partir des valeurs déterminées enregistrées de l'un des au moins deux pics et/ou l'évaluation des informations contenues dans ladite valeur de compensation.

7. Procédé tel que revendiqué selon l'une quelconque des revendications 1 à 5, l'étape de détermination et d'enregistrement (42) de valeurs d'au moins deux pics de la transformée de Fourier $A(\nu)$ calculée comprenant la détermination et l'enregistrement de valeurs d'un pic d'ordre n et de valeurs d'un pic d'ordre -n de la transformée de Fourier $A(\nu)$ calculée, et l'étape de détermination d'au moins un paramètre de caractéristique de respiration du mouvement de respiration du sujet (24) comprenant la détermination d'un retard de phase ($\Phi$) entre des mouvements de respiration de la poitrine et de la région abdominale du sujet en déterminant une différence des fréquences porteuses radar auxquelles des valeurs extrêmes du pic d'ordre n et du pic d'ordre -n apparaissent.

8. Procédé tel que revendiqué selon la revendication 1, l'étape de détermination et d'enregistrement (42) des valeurs d'au moins deux pics de la transformée de Fourier $A(\nu)$ calculée comprenant la détermination et l'enregistrement d'une pluralité d'au moins deux pics additionnels de la transformée de Fourier $A(\nu)$ calculée représentant des harmoniques d'ordre supérieur par rapport à les au moins deux pics, l'étape de détermination (46, 48) d'au moins un paramètre de caractéristique de respiration du mouvement de respiration du sujet (24) comprenant une détermination d'au moins l'une parmi une détermination d'une amplitude de respiration de poitrine et d'une amplitude de respiration de région abdominale, et comprenant en outre une détermination d'une stagnation de la au moins une parmi une amplitude de respiration de poitrine et une amplitude de respiration de région abdominale en comparant des valeurs enregistrées de la pluralité d'au moins deux pics additionnels de la transformée de Fourier $A(\nu)$ calculée à des valeurs seuils prédéterminées pour la pluralité d'au moins deux pics additionnels de la transformée de Fourier $A(\nu)$ calculée, et en détectant que des valeurs enregistrées de la pluralité d'au moins deux pics additionnels de la transformée de Fourier $A(\nu)$ calculée excèdent les valeurs seuils prédéterminées pour une période de temps prédéterminée.

9. Procédé tel que revendiqué selon la revendication 1, l'étape de détermination et d'enregistrement (42) comprenant une détermination et un enregistrement des valeurs d'au moins trois pics de la transformée de Fourier $A(\nu)$ calculée, et l'étape de détermination (46, 48) d'au moins un paramètre de caractéristique de respiration du mouvement de respiration du sujet (24) comprenant la détermination de l'un parmi une pluralité d'intervalles d'amplitude de respiration prédéterminés sur la base de l'exécution d'un classement des valeurs déterminées des au moins trois pics.

10. Procédé tel que revendiqué selon la revendication 7, comprenant en outre des étapes consistant à

- comparer le au moins un déterminé parmi un retard de phase ($\Phi$), une amplitude de respiration de poitrine et une amplitude de respiration de région abdominale à au moins une valeur seuil prédéterminée, et
- générer un signal de sortie représentant un état de respiration du sujet (24).

11. Système capteur radar (10) destiné à surveiller un mouvement de respiration d'un sujet (24), le système capteur radar (10) comprenant

- une unité de transmission radar ayant une antenne de transmission radar (12) et étant conçue pour transmettre des ondes radar (14) à une fréquence porteuse radar ($f_0$, $f$) prédéterminée vers une poitrine (26) et une région abdominale (28) du sujet (24),
- une unité de réception radar ayant une antenne de réception radar (16) et étant conçue pour recevoir des ondes radar (18) qui ont été transmises par l'unité de transmission radar et qui ont été reflétées par le sujet (24), et
- une unité d'évaluation et de commande (22) qui est au moins conçue pour évaluer des informations Doppler provenant des ondes radar (18) reçues par l'unité de réception radar, et qui est en outre conçue pour exécuter automatiquement des étapes du procédé tel que revendiqué selon l'une quelconque des revendications précédentes.

**12.** Système capteur radar (10) tel que revendiqué selon la revendication 11, la fréquence porteuse radar ($f_0$, $f$) prédéterminée reposant dans une plage de fréquences comprise entre 15 GHz et 130 GHz et plus préférablement dans une plage de fréquences comprise entre 57 GHz et 64 GHz.

**13.** Système capteur radar (10) tel que revendiqué selon les revendications 11 ou 12, l'antenne de transmission radar (12) étant conçue pour présenter un lobe principal qui est capable de couvrir une partie majeure de la poitrine (26) et la région abdominale (28) du sujet (24).

**14.** Module logiciel de commande d'exécution automatique du procédé tel que revendiqué selon l'une quelconque des revendications 1 à 10, les étapes de procédé à conduire étant converties en un code de programme du module logiciel, le code de programme pouvant être mis en oeuvre dans une unité mémoire de données numériques du système capteur radar ou une unité de commande séparée et pouvant être exécutée par une unité de processeur du système capteur radar ou une unité de commande séparée, en interface avec le système capteur radar.

Fig. 1

Fig. 2a

Fourier frequency ν

Fig. 2b

Fig. 3

Fig. 4

Region where $a_2 > a_1 > a_3$

$$y = \frac{4\pi x_0}{\lambda}$$

$|J_5(y)|^2$
$|J_4(y)|^2$
$|J_3(y)|^2$
$|J_2(y)|^2$
$|J_1(y)|^2$

Fig. 5

$\phi = \frac{\pi}{6}$

$a_1$

$a_{-1}$

$\Delta f_1$

$$\phi = \pi \frac{\Delta f_1}{\Delta f_{bw}} = \pi \frac{0.4}{2.5} = \pi/6$$

$\phi = \frac{\pi}{3}$

$\Delta f_{bw}$

$a_1$

$a_{-1}$

$\Delta f_1$

$$\phi = \pi \frac{\Delta f_1}{\Delta f_{bw}} = \pi \frac{0.8}{2.5} = \pi/3$$

Fig. 6

Fig. 7

Fig. 8

Fig. 9

Fig. 10

EP 3 641 649 B1

Velocities: "@(x) 2*pi*0.75*0.00468*cos(2*pi*0.75*x)'@(x) 2*pi*0.75*0.00312*cos
(2*pi*0.75*x+pi/4)"'Samp. f:1kHz

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20160200276 A1
- WO 2015140333 A1
- US 20100152600 A1

### Non-patent literature cited in the description

- Infant Respiratory Function Testing. John Wiley & Sons, 1996
- **Y. S. LEE ; P. N. PATHIRANA ; C. L. STEINFORT.** Detection of respiratory paradoxical movement via Doppler radar measurements. *International Conference on Information and Automation for Sustainability,* 1-5
- **TING ZHANG et al.** Non-Contact Estimation at 60 GHz for Human Vital Signs Monitoring Using a Robust Optimization Algorithm. *Conference IEEE APS 2016,* June 2016
- **W. A. GARDNER.** Statistical Spectral Analysis: A Nonprobabilistic Theory. Prentice-Hall, 1987
- **TING ZHANG.** *Non-Contact Estimation at 60 GHz for Human Vital Signs Monitoring Using a Robust Optimization Algorithm*